# EUROPEAN PATENT APPLICATION

(11) **EP 4 748 850 A1**
(43) Date of publication of application: **27.05.2026**
(21) Application number: 25816127.2
(22) Date of filing: 20.02.2025
(51) Int. Cl.: C07K 14/34, C12N 15/77, C12P 13/10

(54) **PROTEIN VARIANT AND L-ARGININE PRODUCTION METHOD USING SAME**

(30) Priority: 31.05.2024 KR 20240071881; 07.10.2024 KR 20240135802
(71) Applicant: CJ Cheiljedang Corporation, Seoul 04560 (KR)
(72) Inventor: KIM, Gyuree, Seoul 04560 (KR); KIM, Ye-Eun, Seoul 04560 (KR); JUNG, Hwi-Min, Seoul 04560 (KR); KIM, Bina, Seoul 04560 (KR); HWANG, Yeji, Seoul 04560 (KR); KIM, Eunji, Seoul 04560 (KR); CHOI, Jin-Geun, Seoul 04560 (KR); SHIM, Jihyun, Seoul 04560 (KR); RHO, Jin Ah, Seoul 04560 (KR)
(74) Representative: Meissner Bolte Partnerschaft mbB
(86) International application number: PCT/KR2025/002418
(87) International publication number: WO 2025/249709

(57) **Abstract**

The present disclosure relates to: a protein variant; a polynucleotide encoding the protein variant; a microorganism of the genus Corynebacterium, comprising at least one selected from the group consisting of the protein variant and the polynucleotide; and an L-arginine production method using the microorganism.

## Description

### [TECHNICAL FIELD]

### CROSS-REFERENCE TO RELATED APPLICATION(S)

This application claims the benefit of priority based on Korean Patent Application No. 10-2024-0071881 filed on May 31, 2024, and Korean Patent Application No. 10-2024-0135802 filed on October 7, 2024, and all contents disclosed in the documents of the corresponding Korean patent applications are incorporated as part of the present specification.

The present disclosure relates to a novel protein variant, a microorganism of the genus Corynebacterium comprising the protein variant, and a method for producing L-arginine using the microorganism.

### [BACKGROUND ART]

L-arginine is a type of amino acid, a basic building block of proteins, and is used as an animal feed additive, food additive, nutritional supplement, and raw material for pharmaceuticals. Furthermore, L-arginine is an amino acid essential for the growth and reproduction of animals, and is widely used in poultry and fish feed. It also promotes muscle formation and waste excretion, leading to increased use as a food-grade amino acid.

Based on these trends, various attempts have been made to improve production ability in a method for producing L-arginine using various microorganisms, including microorganisms of the genus Corynebacterium (US 2016-0145661 A1).

Despite such efforts, development of technology to improve the productivity of L-arginine is still being required.

### [DISCLOSURE]

### [TECHNICAL PROBLEM]

An object of the present disclosure is to provide a novel protein variant.

Another object of the present disclosure is to provide a polynucleotide encoding the protein variant.

Still another object of the present disclosure is to provide a microorganism of the genus *Corynebacterium* comprising at least one selected from the group consisting of the protein variant and a polynucleotide encoding the same.

Still another object of the present disclosure is to provide a method for producing L-arginine, comprising a step of culturing the microorganism in a medium.

Still another object of the present disclosure is to provide a use of the microorganism for producing L-arginine.

### [TECHNICAL SOLUTION]

Description thereof in detail is as follows. Meanwhile, each description and embodiment disclosed in the present disclosure may also be applied to other descriptions and embodiments. That is, all combinations of various elements disclosed in the present disclosure fall within the scope of the present disclosure. In addition, it cannot be considered that the scope of the present disclosure is limited by the specific descriptions described below. Furthermore, those skilled in the art will recognize, or be able to ascertain using no more than routine experimentation, many equivalents to the specific aspects of the present disclosure described in the present disclosure. In addition, such equivalents are intended to be included in the present disclosure.

An aspect of the present disclosure provides a protein variant comprising an amino acid sequence in which an amino acid corresponding to position 922 from the N-terminus in the amino acid sequence of SEQ ID NO: 74 is substituted with an amino acid different from the original amino acid.

In the present disclosure, the sequence of SEQ ID NO: 74 is as shown in Table 1 below.

**[TABLE 1]**

| Protein name (gene ID, (gene name) | Sequence (N-terminus → C-terminus) | SEQ ID NO |
|---|---|---|
| Excinuclease ABC subunit A (BBD29_07445, uvrA) | MADRLVVRGAREHNLKGVDIDLPRDSMVVFTGLSGSGKS SLAFDTIFAEGQRRYVESLSSYARMFLGQMDKPDVDLIDG LSPAVSIDQKSTNRNPRSTVGTITEVYDYLRLLYARAGTAH CPVCDARVERQTPQQMVDQILGMEEGLKFQILAPVVRTR KGEFVDLFADLASQGYSRVRVDGEVHQLSDPPKLEKQIK HDIDVVVDRLQVKASQKQRLTDSMETALRLADGVAVLEF VGLEEDDPNRLRRFSEKMSCPNGHALTVDELEPRAFSFNS PYGACPACDGLGVRTEVDIDLIIPDPDAPATKAVQPWNSSP NHSYFEKLIEGLAKALGFDPETPYSELTAAQKKALVYGSK EEVSVRYKNRYGRVRSWTAPFEGVMGYFDRKLEQTDSET QKDRLLGYTREVPCPTCKGARLKPEILAVRLDSGSHGALS IAGLTALSVHEAFEFLDNLTLGKREEMIAGAVLREIHARLK FLLDVGLSYLTLDRAAGTLSGGEAQRIRLATQIGSGLAGV LYVLDEPSIGLHQRDNQRLITTLEHLRDIGNTLIVVEHDED TIRRADWLVDIGPRAGEFGGEVVYQGEPKGILDCEESLTG AYLSGRRTLGVPDTRREIDKERQLKVVGARENNLRGIDV KIPLGVLCCITGVSGSGKSTLVNQILAKVLANKLNRARQV PGRAKRVEGLEHLDKLVQVDQSPIGRTPRSNPATYTGVFD KVRNLFAETTEAKVRGYKPGRFSFNIKGGRCEACQGDGT LKIEMNFLPDVYVPCEVCDGQRYNRETLEVKYKGKNIAE VLGMPISEAADFFEPITSIHRYLATLVDVGLGYVRLGQAAT TLSGGEAQRVKLAAELQKRSNGRTVYILDEPTTGLHFEDI RKLMMVIQGLVDKGNSVIIIEHNLDVIKAADWIVDMGPE GGSGGGTVVAEGTPEQVAEVAGSYTGQFLKELL | 74 |

In one embodiment, the protein variant of the present disclosure described above may consist essentially of an amino acid sequence in which an amino acid corresponding to position 922 from the N-terminus in the amino acid sequence of SEQ ID NO: 74 is substituted with an amino acid different from the original. In another embodiment, the protein variant of the present disclosure described above may consist of an amino acid sequence in which an amino acid corresponding to position 922 from the N-terminus in the amino acid sequence of SEQ ID NO: 74 is substituted with an amino acid different from the original.

In the present disclosure, the amino acid sequence of SEQ ID NO: 74 may be a sequence of a protein (excinuclease ABC subunit A) encoding by the BBD29_07445 gene.

In one embodiment, the protein variant of the present disclosure described above may be a protein variant comprising an amino acid sequence in which an amino acid corresponding to position 922 from the N-terminus in the amino acid sequence of SEQ ID NO: 74 is substituted with glutamic acid (Glu, E).

In one embodiment, the amino acid corresponding to position 922 from the N-terminus in the amino acid sequence of SEQ ID NO: 74 may be glycine (Gly, G), but is not limited thereto.

The protein variant of the present disclosure may have an activity to increase L-arginine production ability compared to a wild-type protein (polypeptide).

The protein variant of the present disclosure may comprise an amino acid sequence in which an amino acid corresponding to position 922 from the N-terminus in the amino acid sequence of SEQ ID NO: 74 is substituted with an amino acid different from the original amino acid, or an amino acid sequence having at least 70%, 75%, 80%, 85%, 90%, 95%, 96%, 97%, 98%, 99%, 99.5%, 99.7%, or 99.9% or more of homology or identity with the amino acid sequence. In addition, it is obvious that a variant having an amino acid sequence in which a part of the sequence is deleted, modified, substituted, conservatively substituted, or inserted is also included within the scope of the present disclosure, as long as it has such homology or identity and exhibits an efficacy corresponding to the variant of the present disclosure (activity of increasing L-arginine production).

For example, it is a case of having a sequence addition or deletion that does not change the function of the variant of the present disclosure at the N-terminus, C-terminus, and/or internal part of the amino acid sequence, a naturally occurring mutation, a silent mutation, or a conservative substitution.

The "conservative substitution" means substituting one amino acid with another amino acid having similar structural and/or chemical properties. Such amino acid substitutions may generally occur based on similarities in the polarity, charge, solubility, hydrophobicity, hydrophilicity, and/or amphipathic nature of the residues. Typically, conservative substitutions may have little or no effect on the activity of a protein or polypeptide.

In the present disclosure, the term "variant" refers to a polypeptide in which one or more amino acids are conservatively substituted and/or modified to be different from the amino acid sequence before variation of the variant, but whose functions or properties are maintained. Such variants may generally be identified by modifying one or more amino acids in the amino acid sequence of the polypeptide and evaluating the properties of the modified polypeptide. That is, the ability of the variant may be increased, unchanged, or decreased compared to the polypeptide before variation. In addition, some variants may include variants in which one or more parts such as an N-terminal leader sequence or a transmembrane domain are deleted. Other variants may include variants in which a portion is removed from the N- and/or C-terminus of a mature protein. The term "variant" may be used interchangeably with terms such as variant, modification, variant polypeptide, mutated protein, mutation, and variant (as English expressions, modification, modified polypeptide, modified protein, mutant, mutein, divergent, etc.), and is not limited thereto as long as the term is used in the sense of being mutated.

In addition, the variant may comprise deletions or additions of amino acids having minimal influence on the properties and secondary structure of the polypeptide. For example, a signal (or leader) sequence involved in the translocation of the protein co-translationally or post-translationally may be conjugated to the N-terminus of the variant. In addition, the variant may be conjugated with another sequence or a linker so as to be identified, purified, or synthesized.

In one embodiment, the protein variant of the present disclosure described above may have 90%, 91%, 93%, 94%, 95%, 96%, 97%, 98%, or 99% or more and less than 100% of sequence homology or identity with the amino acid sequence of SEQ ID NO: 74.

Another aspect of the present disclosure provides a protein variant comprising any one amino acid sequence selected from the group consisting of the amino acid sequences of SEQ ID NO: 15 to SEQ ID NO: 20. In one embodiment, the protein variant may be a protein variant comprising the amino acid sequence of SEQ ID NO: 20. In another embodiment, the variant of the present disclosure may consist essentially of the amino acid sequence of SEQ ID NO: 20. In still another embodiment, the variant of the present disclosure may consist of the amino acid sequence of SEQ ID NO: 20.

In the present disclosure, the amino acid sequence of SEQ ID NO: 20 may be a sequence of a protein variant in which the 922nd amino acid of a protein (excinuclease ABC subunit A) encoded by the BBD29_07445 gene is substituted from glycine (Gly, G) to glutamic acid (Glu, E).

The protein variant of the present disclosure may comprise any one amino acid sequence selected from the group consisting of the amino acid sequences of SEQ ID NO: 15 to SEQ ID NO: 20 (for example, the amino acid sequence of SEQ ID NO: 20), or may comprise an amino acid sequence having at least 70%, 75%, 80%, 85%, 90%, 95%, 96%, 97%, 98%, 99%, 99.5%, 99.7%, or 99.9% or more homology or identity with the amino acid sequence. In addition, it is obvious that a variant having an amino acid sequence in which a part of the sequence is deleted, modified, substituted, conservatively substituted or added is also included within the scope of the present disclosure, as long as it has such homology or identity and exhibits an efficacy (activity of increasing L-arginine production) corresponding to the variant of the present disclosure.

For example, it is a case of having sequence additions or deletions, naturally occurring mutations, silent mutations, or conservative substitutions that do not alter the function of the variant of the present disclosure at the N-terminus, C-terminus, and/or interior of the amino acid sequence.

In the protein variant, conservative substitutions, variants, and the like are as described above.

In the present disclosure, the term, 'homology' or 'identity' means the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and default gap penalties established by the program used may be employed together. Substantially, homologous or identical sequences can generally hybridize with the entire sequence or a portion thereof under medium or high stringent conditions. It is apparent that hybridization also includes hybridization with a polynucleotide containing a general codon or codon by considering the codon degeneracy in the polynucleotide.

Whether any two polynucleotide or polypeptide sequences have homology or identity may be determined, for example, by using a known computer algorithm such as the "FASTA" program using default parameters as in Pearson et al (1988) [Proc.Natl. Acad. Sci. USA 85]: 2444. Alternatively, the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or a later version), may be used for the determination (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073)). For example, homology or identity may be determined using BLAST of the National Center for Biotechnology Information (NCBI) database, or ClustalW.

The homology or identity of a polynucleotide or a polypeptide may be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program can be defined as the value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for end gaps.

As an example of the present disclosure, the variant of the present disclosure described above may have an activity increasing L-arginine production ability, compared to a wild type protein (polypeptide).

In the present disclosure, the term "corresponding to" refers to an amino acid residue at a position enumerated in a polypeptide, or an amino acid residue that is similar to, identical to, or homologous to a residue enumerated in a polypeptide. Identifying an amino acid at a corresponding position may be determining a specific amino acid of a sequence with reference to a specific sequence. As used in the present disclosure, "corresponding region" generally refers to a similar or corresponding position in a related protein or a reference protein.

For example, an arbitrary amino acid sequence is aligned with an amino acid sequence in which an amino acid corresponding to position 922 from the N-terminus in the amino acid sequence of SEQ ID NO: 74 is substituted with an amino acid different from the original (for example, the amino acid sequence of SEQ ID NO: 20), and based on this, each amino acid residue of the amino acid sequence can be numbered by referring to the numerical position of the amino acid residue corresponding to the amino acid residue of the amino acid sequence in which the amino acid corresponding to position 922 from the N-terminus in the amino acid sequence of SEQ ID NO: 74 is substituted with an amino acid different from the original (for example, the amino acid sequence of SEQ ID NO: 20). For example, a sequence alignment algorithm as described in the present disclosure can identify positions of amino acids or positions where modifications such as substitution, insertion, or deletion occur by comparing it with a query sequence (also referred to as a "reference sequence").

For such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), etc. can be used, but are not limited thereto, and sequence alignment programs, pairwise sequence comparison algorithms, etc., known in the art can be appropriately used.

Another aspect of the present disclosure is to provide a polynucleotide encoding the protein variant of the present disclosure.

In the present disclosure, the term "polynucleotide" refers to a polymer of nucleotides in which nucleotide monomers are connected in a long chain shape by covalent bonds, which is a DNA or RNA strand of a certain length or more, and more specifically refers to a polynucleotide fragment encoding the variant.

In one example, the polynucleotide may be a polynucleotide encoding a protein variant comprising an amino acid sequence in which an amino acid corresponding to position 922 from the N-terminus in the amino acid sequence of SEQ ID NO: 74 is substituted with an amino acid different from the original.

In one example, the polynucleotide may be a polynucleotide comprising any one nucleotide sequence selected from the group consisting of the nucleotide sequences of SEQ ID NO: 21 to SEQ ID NO: 26, for example, the nucleotide sequence of SEQ ID NO: 26.

The polynucleotide encoding the protein variant of the present disclosure may have or comprise any one nucleotide sequence selected from the group consisting of the nucleotide sequences of SEQ ID NO: 21 to SEQ ID NO: 26, for example, the nucleotide sequence of SEQ ID NO: 26. In another example, the polynucleotide may comprise a nucleotide sequence having at least 70%, 75%, 80%, 85%, 90%, 95%, 96%, 97%, 98%, 99%, 99.5%, 99.7%, or 99.9% or more homology or identity with the nucleotide sequence. In addition, it is obvious that a polynucleotide having a nucleotide sequence in which a portion of the sequence is deleted, modified, substituted, conservatively substituted, or inserted is also included within the scope of the present disclosure as long as it is a sequence encoding a polypeptide or protein that has such homology or identity and exhibits an activity (increase in L-arginine production) corresponding to the variant of the present disclosure.

In one example, the nucleotide sequence or amino acid sequence provided in the present disclosure may include those modified by conventional mutagenesis methods, for example, directed evolution and/or site-directed mutagenesis, to the extent that their original functions or desired functions are maintained. In one example, that a polynucleotide or polypeptide "comprising a specific nucleotide sequence or amino acid sequence" may mean that the polynucleotide or polypeptide (i) consists of or essentially includes the specific nucleotide sequence or amino acid sequence, or (ii) consists of or essentially includes an amino acid sequence having 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more homology with the specific nucleotide sequence or amino acid sequence and maintains the original function and/or desired function.

The polynucleotide of the present disclosure may undergo various modifications in the coding region within a range that does not change the amino acid sequence of the variant of the present disclosure, considering the degeneracy of codons or codons preferred in an organism in which the variant of the present disclosure is to be expressed. Specifically, the polynucleotide of the present disclosure may have or include a base sequence with 70% or more, 75% or more, 80% or more, 85% or more, 90% or more, 95% or more, 96% or more, 97% or more, 98% or more, and less than 100% homology or identity with any one nucleotide sequence (e.g., the nucleotide sequence of SEQ ID NO: 26) selected from the group consisting of the nucleotide sequences of SEQ ID NO: 21 to SEQ ID NO: 26, or may consist of or essentially consist of a base sequence with 70% or more, 75% or more, 80% or more, 85% or more, 90% or more, 95% or more, 96% or more, 97% or more, 98% or more, and less than 100% homology or identity with the nucleotide sequence, but is not limited thereto.

In addition, the polynucleotide of the present disclosure may comprise, without limitation, a probe that can be prepared from a known gene sequence, for example, a sequence that can hybridize under stringent conditions with a complementary sequence to the whole or a part of the polynucleotide sequence of the present disclosure. The "stringent condition" means a condition that enables specific hybridization between polynucleotides. For example, conditions in which polynucleotides with high homology or identity, specifically polynucleotides having 70% or more, 75% or more, 80% or more, 85% or more, 90% or more, 95% or more, 96% or more, 97% or more, 98% or more, or 99% or more of homology or identity, hybridize with each other, while polynucleotides with lower homology or identity do not hybridize with each other, or conditions of washing once, specifically 2 to 3 times, at a salt concentration and temperature corresponding to 60°C, 1×SSC, 0.1% SDS, specifically 60°C, 0.1×SSC, 0.1% SDS, more specifically 68°C, 0.1×SSC, 0.1% SDS, which are washing conditions for conventional Southern hybridization, can be listed.

Hybridization requires that two nucleic acids have complementary sequences, even though mismatches between bases are possible depending on the stringency of hybridization. The term "complementary" is used to describe the relationship between nucleotide bases that can hybridize with each other. For example, regarding DNA, adenine is complementary to thymine and cytosine is complementary to guanine. Accordingly, the polynucleotide of the present disclosure may also comprise not only substantially similar nucleotide sequences but also isolated nucleic acid fragments complementary to the entire sequence.

Specifically, a polynucleotide having homology or identity with the polynucleotide of the present disclosure can be detected using hybridization conditions including a hybridization step at a Tm value of 55°C and using the conditions described above. In addition, the Tm value may be 60°C, 63°C, or 65°C, but is not limited thereto and may be appropriately adjusted by those skilled in the art according to the purpose.

The appropriate stringency for hybridizing the polynucleotide depends on the length and degree of complementarity of the polynucleotide, and the variables are well known in the technical field.

Another aspect of the present disclosure is to provide a vector comprising the polynucleotide of the present disclosure. The vector may be an expression vector for expressing the polynucleotide in a host cell, but is not limited thereto.

The vector of the present disclosure may comprise a DNA construct comprising a base sequence of a polynucleotide encoding a target polypeptide operably linked to a suitable expression control region (or expression control sequence) so that the target polypeptide can be expressed in a suitable host. The expression control region may comprise a promoter capable of initiating transcription, any operator sequence for regulating such transcription, a sequence encoding a suitable mRNA ribosome binding site, and a sequence for regulating the termination of transcription and translation. After being transformed into a suitable host cell, the vector may replicate or function independently of the host genome, or may be integrated into the genome itself.

The vector used in the present disclosure is not particularly limited, and any vector known in the art may be used. Examples of commonly used vectors include plasmids, cosmids, viruses, and bacteriophages in a natural state or a recombinant state. For example, as a phage vector or a cosmid vector, pWE15, M13, MBL3, MBL4, IXII, ASHII, APII, t10, t11, Charon4A, Charon21A, etc., may be used; and as a plasmid vector, pDZ-based, pBR-based, pUC-based, pBluescriptII-based, pGEM-based, pTZ-based, pCL-based, pET-based vectors, etc., may be used. Specifically, pDZ, pDC, pDCM2, pACYC177, pACYC184, pCL, pECCG117, pUC19, pBR322, pMW118, pCC1BAC, or pDC24 vectors, etc., may be used.

As an example, a polynucleotide encoding a target polypeptide can be inserted into a chromosome through a vector for intracellular chromosomal insertion. The insertion of the polynucleotide into the chromosome may be performed by any method known in the art, for example, by homologous recombination, but is not limited thereto. A selection marker for confirming whether the chromosome is inserted may be further included. The selection marker is for selecting cells transformed with a vector, that is, for confirming whether a target nucleic acid molecule has been inserted, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means introducing a vector comprising a polynucleotide encoding a target polypeptide into a host cell or a microorganism so that the polypeptide encoded by the polynucleotide can be expressed in the host cell. The transformed polynucleotide may include all of those regardless of whether it is located by being inserted into the chromosome of the host cell or located outside the chromosome, as long as it can be expressed in the host cell. In addition, the polynucleotide includes DNA and/or RNA encoding a target polypeptide. The polynucleotide may be introduced in any form as long as it can be introduced into and expressed in a host cell. For example, the polynucleotide may be introduced into a host cell in the form of an expression cassette, which is a gene construct containing all elements necessary for its own expression. The expression cassette may typically comprise a promoter, a transcription termination signal, a ribosome binding site, and a translation termination signal operably linked to the polynucleotide. The expression cassette may be in the form of a self-replicable expression vector. In addition, the polynucleotide may be introduced into a host cell in its own form and operably linked to a sequence required for expression in the host cell, but is not limited thereto.

In addition, the term "operably linked" above means that a promoter sequence is functionally linked to the polynucleotide sequence so as to initiate and mediate transcription of the polynucleotide encoding a target variant of the present disclosure.

Another aspect of the present disclosure provides a microorganism of the genus *Corynebacterium* comprising at least one selected from the group consisting of a protein variant of the present disclosure and a polynucleotide of the present disclosure.

The microorganism of the present disclosure may comprise a protein variant of the present disclosure, a polynucleotide encoding the protein variant, or a vector comprising a polynucleotide of the present disclosure.

In the present disclosure, the term "microorganism (or strain)" includes all of wild-type microorganisms or microorganisms in which genetic modification occurs naturally or artificially, and may be a microorganism of which specific mechanism is weakened or strengthened due to reasons such as insertion of a foreign gene or enhancement or inactivation of activity of an endogenous gene and the like, which is a microorganism comprising genetic modification for production of a target polypeptide, protein or product.

The microorganism of the present disclosure may be a microorganism comprising any one or more of a protein variant of the present disclosure, a polynucleotide of the present disclosure, and a vector comprising a polynucleotide of the present disclosure; a microorganism modified to express the variant of the present disclosure or a polynucleotide of the present disclosure; a microorganism expressing a variant of the present disclosure or a polynucleotide of the present disclosure (e.g., a recombinant microorganism); or a microorganism having the activity of a variant of the present disclosure (e.g., a recombinant microorganism), but is not limited thereto.

The microorganism of the present disclosure may be a microorganism having L-arginine production ability.

The microorganism of the present disclosure may have increased L-arginine production ability compared to a microorganism of the genus *Corynebacterium* that does not comprise at least one selected from the group consisting of the protein variant of the present disclosure described above and a polynucleotide encoding the protein variant (that is, a protein variant comprising an amino acid sequence in which an amino acid corresponding to position 922 from the N-terminus in the amino acid sequence of SEQ ID NO: 74 is substituted with glutamic acid; or a microorganism that does not express a protein variant comprising any one amino acid sequence selected from the group consisting of amino acid sequences of SEQ ID NO: 15 to SEQ ID NO: 20, for example, the amino acid sequence of SEQ ID NO: 20, or a microorganism that expresses a wild-type protein corresponding to the protein variant).

The microorganism of the present disclosure may be a microorganism naturally having L-arginine production ability, or a microorganism in which a variant of the present disclosure or a polynucleotide encoding the same (or a vector comprising the polynucleotide) is introduced into a parent strain not having L-arginine production ability and/or a microorganism to which L-arginine production ability is conferred, but is not limited thereto.

As an example, the microorganism of the present disclosure is a cell or microorganism expressing a protein variant of the present disclosure by being transformed with a polynucleotide of the present disclosure or a vector comprising a polynucleotide encoding a variant of the present disclosure, and for the purpose of the present disclosure, the strain of the present disclosure may include all microorganisms capable of producing L-arginine including a variant of the present disclosure. For example, the microorganism of the present disclosure may be a recombinant microorganism having increased L-arginine production ability by introducing a polynucleotide encoding a variant of the present disclosure into a natural wild-type microorganism or a microorganism producing L-arginine.

In one specific embodiment, the microorganism of the present disclosure may have increased L-arginine production ability compared to a microorganism of the genus *Corynebacterium* that does not comprise at least one selected from the group consisting of a protein variant of the present disclosure and a polynucleotide encoding the protein variant. The microorganism of the genus *Corynebacterium* that does not comprise at least one selected from the group consisting of the protein variant of the present disclosure and the polynucleotide encoding the protein variant may be a natural wild-type microorganism or a non-modified microorganism, and may also be expressed as a parent strain.

In the present disclosure, a "non-modified microorganism" does not exclude a microorganism including a naturally occurring mutation, and may refer to a wild-type microorganism or a naturally-occurring strain itself, or a microorganism before its properties are changed by genetic variation due to natural or artificial factors. For example, the non-modified microorganism may refer to a microorganism in which the protein variant described in the present disclosure has not been introduced, or a strain before such introduction. The "non-modified microorganism" may be used interchangeably with "pre-modification strain", "pre-modification microorganism", "non-mutant strain", " non-modified strain", "non-mutant microorganism", or "reference microorganism".

In one embodiment, the unmodified microorganism may be a microorganism in which the biosynthetic pathway of L-arginine is additionally enhanced to increase the production of L-arginine, but is not limited thereto.

In one embodiment, in order to enhance the biosynthetic pathway of L-arginine, the non-modified microorganism may be, for example, a microorganism in which an argR gene encoding an Arginine repressor (ArgR) is deleted, a genetic mutation (M54V) is introduced into an argB gene encoding an Acetylglutamate kinase (ArgB), and/or an activity of N-acetyl-gamma-glutamyl-phosphate reductase (ArgC) is reinforced by promoter replacement, but is not limited thereto.

In one embodiment, the microorganism with increased L-arginine production of the present disclosure may be one in which L-arginine production capacity is increased by about 1% or more, about 1.5% or more, about 2% or more, about 2.5% or more, about 3% or more, about 3.5% or more, about 4% or more, about 4.5% or more, about 5% or more, about 5.5% or more, about 6% or more, or about 6.1% or more (the upper limit is not particularly limited and may be, for example, about 200% or less, about 150% or less, about 100% or less, or about 50% or less) compared to a parent strain before mutation or an unmodified microorganism, but is not limited thereto. In another embodiment, the microorganism with increased L-arginine production of the present disclosure may be one in which L-arginine production capacity is increased by about 1.01 times or more, about 1.015 times or more, about 1.02 times or more, about 1.025 times or more, about 1.03 times or more, about 1.035 times or more, about 1.04 times or more, about 1.045 times or more, about 1.05 times or more, about 1.055 times or more, about 1.06 times or more, or about 1.061 times or more (the upper limit is not particularly limited and may be, for example, about 10 times or less, about 5 times or less, about 3 times or less, or about 2 times or less) compared to a parent strain before mutation or a non-modified microorganism, but is not limited thereto.

The term "about" is a range including all of ±0.5, ±0.4, ±0.3, ±0.2, ±0.1, and the like, and includes all numerical values identical or similar to the numerical value appearing after the term of about, but is not limited thereto.

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium pollutisoli, Corynebacterium imitans, Corynebacterium testudinoris, or Corynebacterium flavescens,* and more specifically, may be *Corynebacterium glutamicum.*

In the present disclosure, the term, "weakening" of the polypeptide, is a concept including both reducing activity or having no activity compared to the endogenous activity. The weakening may be interchangeably used with terms such as inactivation, deficiency, down-regulation, decrease, reduce, attenuation, and the like.

The weakening may also include cases where the activity of the polypeptide itself is reduced or removed compared to activity of the polypeptide possessed by the original microorganism due to mutation of a polynucleotide encoding the polypeptide and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "endogenous activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the endogenous activity, refers to that it is lowered compared to the activity of a specific polypeptide originally possessed by a parent strain before trait changes or non-modified microorganism.

This weakening of the activity of the polypeptide may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide of the present disclosure may be by:
1) deletion of all or part of the gene encoding the polypeptide;
2) modification of an expression-control region (or expression-control sequence) such that expression of the gene encoding the polypeptide is reduced;
3) modification of an amino acid sequence constituting the polypeptide such that activity of the polypeptide is eliminated or weakened (e.g., deletion/substitution/addition of one or more amino acids in the amino acid sequence);
4) modification of a gene sequence encoding the polypeptide such that activity of the polypeptide is eliminated or weakened (e.g., deletion/substitution/addition of one or more nucleobases in a nucleobase sequence of the polypeptide gene to encode a modified polypeptide such that the activity of the polypeptide is eliminated or weakened);
5) modification of a nucleotide sequence encoding a start codon or a 5'-UTR region of a gene transcript encoding the polypeptide;
6) introduction of an antisense oligonucleotide (e.g., antisense RNA) that complementarily binds to a transcript of the gene encoding the polypeptide;
7) addition of a sequence complementary to a Shine-Dalgarno sequence to the front end of the Shine-Dalgarno sequence of the gene encoding the polypeptide in order to form a secondary structure to which ribosomes cannot attach;
8) addition of a promoter transcribed in the opposite direction (Reverse transcription engineering, RTE) to the 3' end of an ORF (open reading frame) of the gene sequence encoding the polypeptide; or
9) regulation of the cellular localization of the protein (polypeptide); or
10) a combination of two or more selected from the above 1) to 9), but is not particularly limited thereto.

For example,
the 1) deletion of all or part of the gene encoding the polypeptide may be removal of the entire polynucleotide encoding the endogenous target polypeptide in the chromosome, substitution with a polynucleotide in which some nucleotides are deleted, or substitution with a selection marker gene.

In addition, the 2) modification of the expression control region (or expression control sequence) may be the occurrence of a mutation in the expression control region (or expression control sequence) through deletion, insertion, non-conservative or conservative substitution, or a combination thereof, or substitution with a sequence having weaker activity. The expression control region includes, but is not limited to, a promoter, an operator sequence, a sequence encoding a ribosome binding site, and a sequence regulating termination of transcription and translation.

In addition, the 3) modification of the nucleotide sequence encoding a start codon or 5'-UTR region of the gene transcript encoding the polypeptide may be, for example, substitution with a nucleotide sequence encoding another start codon having a lower polypeptide expression rate compared to the endogenous start codon, but is not limited thereto.

In addition, the modification of the amino acid sequence or polynucleotide sequence of the above 4) and 5) may be the occurrence of a mutation in the sequence through deletion, insertion, non-conservative or conservative substitution, or a combination thereof, or substitution with an amino acid sequence or polynucleotide sequence improved to have weaker activity, or an amino acid sequence or polynucleotide sequence improved to have no activity, so as to weaken the activity of the polypeptide, but is not limited thereto. For example, gene expression may be inhibited or weakened by introducing a mutation in the polynucleotide sequence to form a stop codon, but is not limited thereto.

For the 6) introduction of an antisense oligonucleotide (for example, antisense RNA) that complementarily binds to the transcript of the gene encoding the polypeptide, reference may be made to, for example, the document [Weintraub, H. et al., Antisense-RNA as a molecular tool for genetic analysis, Reviews - Trends in Genetics, Vol. 1(1) 1986].

The 7) addition of a sequence complementary to the Shine-Dalgarno sequence to the front end of the Shine-Dalgarno sequence of the gene encoding the polypeptide in order to form a secondary structure to which ribosomes cannot attach may be to disable mRNA translation or reduce the rate thereof.

The 8) addition of a promoter transcribed in the opposite direction (Reverse transcription engineering, RTE) to the 3' end of the ORF (open reading frame) of the gene sequence encoding the polypeptide may be to weaken the activity by preparing an antisense nucleotide complementary to the transcript of the gene encoding the polypeptide.

The 9) regulation of cellular localization of the protein (polypeptide) may be targeting the protein (polypeptide) to a specific intracellular organelle or a specific intracellular space. For example, it may be targeting to the periplasm or cytoplasm through addition or removal of a leader sequence functioning for targeting the protein (polypeptide), but is not limited thereto.

Such weakening of polypeptide activity may be that the activity or concentration expression level of the corresponding polypeptide is weakened based on the activity or concentration of the polypeptide expressed in a wild-type or microorganism strain before modification, or that the amount of a product produced from the polypeptide is reduced, but is not limited thereto.

In the present disclosure, the term, "enhancement" of the polypeptide activity, means that the activity of the polypeptide is increased compared to the endogenous activity. The enhancement may be interchangeably used with terms such as activation, up-regulation, overexpression, increase and the like. Herein, the activation, up-regulation, overexpression, and increase may include showing activity which is not originally present, or showing activity improved compared to the endogenous activity or activity before modification. The "endogenous activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the endogenous activity, refers to that it is improved compared to the activity and/or concentration (expression level) of a specific polypeptide originally possessed by a parent strain before trait changes or non-modified microorganism.

The enhancement can be achieved by introducing an exogenous polypeptide or through activity enhancement and/or concentration (expression level) of an endogenous polypeptide. Whether the activity of the polypeptide is enhanced can be confirmed from an increase in the degree of activity, expression level of the corresponding polypeptide, or the amount of a product discharged from the polypeptide.

Various methods well known in the art can be applied to the enhancement of the activity of the polypeptide, and it is not limited as long as the activity of the target polypeptide can be enhanced more than that of the microorganism before modification. Specifically, it may be by using gene engineering and/or protein engineering well known to those skilled in the art, which are routine methods of molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be by:
1) increasing of the intracellular copy number of a polynucleotide encoding the polypeptide;
2) replacing of a gene expression regulatory region on a chromosome encoding the polypeptide with a sequence having strong activity;
3) modification of a nucleotide sequence encoding a start codon or a 5'-UTR region of a gene transcript encoding the polypeptide;
4) modification of an amino acid sequence of the polypeptide such that the polypeptide activity is enhanced;
5) modification of a polynucleotide sequence encoding the polypeptide such that the polypeptide activity is enhanced (e.g., modification of the polynucleotide sequence of the polypeptide gene to encode a polypeptide modified such that the activity of the polypeptide is enhanced);
6) introduction of an exogenous polypeptide exhibiting the activity of the polypeptide or an exogenous polynucleotide encoding the same;
7) codon optimization of a polynucleotide encoding the polypeptide;
8) selection and modification of an exposed site or chemical modification by analyzing the tertiary structure of the polypeptide; or
9) regulation of the cellular localization of the protein (polypeptide); or
10) a combination of two or more selected from the above 1) to 9), but is not particularly limited thereto.

The 1) increase of the intracellular copy number of the polynucleotide encoding the polypeptide may be achieved by introducing into a host cell a recombinant vector, which is operably linked to the polynucleotide encoding the polypeptide and can replicate and function independently of the host. Alternatively, it may be achieved by introducing one copy or two or more copies of the polynucleotide encoding the polypeptide into a chromosome within the host cell. The introduction into the chromosome may be performed by introducing a vector capable of inserting the polynucleotide into the chromosome in the host cell into the host cell, but is not limited thereto. The vector is as described above.

The 2) replacement of the gene expression control region (or expression control sequence) on the chromosome encoding the polypeptide with a sequence having strong activity may be, for example, the occurrence of a mutation in the sequence through deletion, insertion, non-conservative or conservative substitution, or a combination thereof to further enhance the activity of the expression control region, or replacement with a sequence having stronger activity. The expression control region is not particularly limited thereto, but may include a promoter, an operator sequence, a sequence encoding a ribosome binding site, and a sequence regulating termination of transcription and translation. As an example, the original promoter may be replaced with a strong promoter, but it is not limited thereto.

Examples of known strong promoters include CJ1 to CJ7 promoters (U.S. Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent No. US 10584338 B2), O2 promoter (U.S. Patent No. US 10273491 B2), tkt promoter, yccA promoter, etc., but are not limited thereto.

The 3) modification of the nucleotide sequence encoding the start codon or the 5'-UTR region of the gene transcript encoding the polypeptide may be, for example, substitution with a nucleotide sequence encoding another start codon having a higher polypeptide expression rate compared to the endogenous start codon, but is not limited thereto.

The modification of the amino acid sequence or polynucleotide sequence of the above 4) and 5) may be the occurrence of a mutation in the sequence through deletion, insertion, non-conservative or conservative substitution, or a combination thereof, or replacement with an amino acid sequence or polynucleotide sequence improved to have stronger activity, or an amino acid sequence or polynucleotide sequence improved to have increased activity, so as to enhance the activity of the polypeptide, but is not limited thereto. The replacement may be specifically performed by inserting the polynucleotide into the genome by homologous recombination, but is not limited thereto. At this time, the vector used may further include a selection marker for confirming whether it has been inserted into the chromosome. The selection marker is as described above.

The 6) introduction of an exogenous polynucleotide exhibiting the activity of the polypeptide may be the introduction into a host cell of an exogenous polynucleotide encoding a polypeptide exhibiting the same/similar activity as the polypeptide. The exogenous polynucleotide is not limited in its origin or sequence as long as it exhibits the same/similar activity as the polypeptide. The method used for the introduction may be performed by appropriately selecting a known transformation method by those skilled in the art, and the polypeptide is produced by expressing the introduced polynucleotide in the host cell, thereby increasing its activity.

The 7) codon optimization of the polynucleotide encoding the polypeptide may be codon optimization of an endogenous polynucleotide to increase transcription or translation in the host cell, or codon optimization of an exogenous polynucleotide to achieve optimized transcription and translation in the host cell.

The 8) selection and modification of an exposed site or chemical modification by analyzing the tertiary structure of the polypeptide may be, for example, determining a template protein candidate according to the degree of sequence similarity by comparing sequence information of the polypeptide to be analyzed with a database in which sequence information of known proteins is stored, identifying the structure based on the same, and selecting and modifying or chemically modifying an exposed site to be modified or chemically modified.

The 9) regulation of cellular localization of the protein (polypeptide) may be targeting the protein (polypeptide) to a specific intracellular organelle or a specific intracellular space. For example, it may be targeting to the periplasm or cytoplasm through addition or removal of a leader sequence functioning for targeting the protein (polypeptide), but is not limited thereto.

Such enhancement of polypeptide activity may be that the activity or concentration expression level of the corresponding polypeptide is increased based on the activity or concentration of the polypeptide expressed in a wild-type or microorganism strain before modification, or that the amount of a product produced from the polypeptide is increased, but is not limited thereto.

Modification of part or all of a polynucleotide in the microorganism of the present disclosure (e.g., modification for encoding the above-described protein variant) may be induced by (a) genome editing using homologous recombination or an engineered nuclease (e.g., CRISPR-Cas9) using a vector for chromosomal insertion in the microorganism and/or (b) treatment with light such as ultraviolet rays and radiation and/or chemical substances, but is not limited thereto. The method for modifying part or all of the gene may include a method by DNA recombination technology. For example, deletion of part or all of a gene can be achieved by injecting a nucleotide sequence or a vector containing a nucleotide sequence having homology with a target gene into the microorganism to cause homologous recombination. The injected nucleotide sequence or vector may include a dominant selection marker, but is not limited thereto.

In the microorganism of the present disclosure, protein variant, polynucleotide, and L-arginine, etc., are as described in the other aspects above.

Another aspect of the present disclosure provides a method for producing L-arginine comprising a step of culturing a microorganism of the genus *Corynebacterium* comprising the protein variant of the present disclosure or the polynucleotide of the present disclosure in a medium.

The method for producing L-arginine of the present disclosure may include a step of culturing a microorganism of the genus *Corynebacterium* comprising the protein variant of the present disclosure or the polynucleotide of the present disclosure or the vector of the present disclosure in a medium.

The term "culturing" in the present disclosure means growing a microorganism of *Corynebacterium* genus under appropriately and artificially controlled environmental conditions. The culturing process of the present disclosure may be performed according to a suitable medium and culture conditions known in the art. Such a culturing process can be easily adjusted and used by a person skilled in the art according to a selected strain. Specifically, the culturing may be a batch, continuous, and/or fed-batch culture, but is not limited thereto.

In the present disclosure, the term "medium" means a substance prepared by mixing nutrients required for culturing the microorganism of the genus *Corynebacterium* of the present disclosure as main components, and supplies water, which is essential for survival and growth, as well as nutrients and growth factors. Specifically, as the medium and other culture conditions used for culturing the microorganism of the genus *Corynebacterium* of the present disclosure, any medium used for culturing conventional microorganisms can be used without particular limitation, but the a microorganism of the genus *Corynebacterium* of the present disclosure can be cultured in a conventional medium containing a suitable carbon source, nitrogen source, phosphorus source, inorganic compound, amino acid and/or vitamin, while controlling temperature, pH, etc., under aerobic conditions.

Specifically, a culture medium for a microorganism of the genus *Corynebacterium* can be found in literature such as ["Manual of Methods for General Bacteriology" by the American Society for Bacteriology (Washington D.C., USA, 1981)].

In the present disclosure, the carbon source may include carbohydrates such as glucose, saccharose, lactose, fructose, sucrose, maltose, etc.; sugar alcohols such as mannitol, sorbitol, etc.; organic acids such as pyruvic acid, lactic acid, citric acid, etc.; and amino acids such as glutamic acid, methionine, lysine, etc. In addition, natural organic nutrient sources such as starch hydrolysate, molasses, blackstrap molasses, rice bran, cassava, sugar cane bagasse, and corn steep liquor can be used, specifically carbohydrates such as glucose and sterilized pretreated molasses (i.e., molasses converted to reducing sugar), etc., can be used, and various other appropriate amounts of carbon sources can be used without limitation. These carbon sources may be used alone or in combination of two or more, and are not limited thereto.

The nitrogen source may include inorganic nitrogen sources such as ammonia, ammonium sulfate, ammonium chloride, ammonium acetate, ammonium phosphate, ammonium carbonate, ammonium nitrate, etc.; and organic nitrogen sources such as amino acids such as glutamic acid, methionine, glutamine, etc., peptone, NZ-amine, meat extract, yeast extract, malt extract, corn steep liquor, casein hydrolysate, fish or a decomposition product thereof, defatted soybean cake or a decomposition product thereof, and the like. These nitrogen sources may be used alone or in combination of two or more, and are not limited thereto.

The phosphorus source may include potassium phosphate monobasic, potassium phosphate dibasic, or a sodium-containing salt corresponding thereto. As the inorganic compound, sodium chloride, calcium chloride, iron chloride, magnesium sulfate, iron sulfate, manganese sulfate, calcium carbonate, etc., may be used, and in addition, amino acids, vitamins, and/or appropriate precursors, etc., may be included. These components or precursors may be added to the medium in a batch or continuous manner. However, it is not limited thereto.

In addition, during culturing of the microorganism of the genus Corynebacterium of the present disclosure, compounds such as ammonium hydroxide, potassium hydroxide, ammonia, phosphoric acid, sulfuric acid, etc., can be added to the medium in an appropriate manner to adjust the pH of the medium. In addition, during culturing, an antifoaming agent such as fatty acid polyglycol ester can be used to suppress foam generation. In addition, to maintain the aerobic state of the medium, oxygen or oxygen-containing gas may be injected into the medium, or to maintain anaerobic and microaerobic states, gas may not be injected or nitrogen, hydrogen, or carbon dioxide gas may be injected, but is not limited thereto.

In the culturing of the present disclosure, the culture temperature may be 20°C to 45°C, specifically 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, but is not limited thereto. In the culturing of the present disclosure, the culturing time may be for about 10 to 160 hours, but is not limited thereto.

L-arginine produced by the culturing of the present disclosure may be secreted into the medium or retained within the cells.

The method of producing L-arginine of the present disclosure may further include, for example, prior to the step of culturing, a step of preparing the microorganism of the genus Corynebacterium of the present disclosure, a step of preparing a medium for culturing the strain, or a combination thereof (in any order).

The method of producing L-arginine of the present disclosure may further comprise a step of recovering L-arginine from the medium (medium in which culturing was performed) or the microorganism of the genus *Corynebacterium* according to the culturing. The step of recovering may be further included after the step of culturing.

The recovering may be to collect the target product of L-arginine using a suitable method known in the pertinent art, according to the culturing method, for example, a batch, continuous, or fed-batch culturing method. For example, various types of chromatography such as centrifugation, filtration, treatment with a crystallizing protein precipitating agent (salting out), extraction, ultrasonic disruption, ultrafiltration, dialysis, molecular sieve chromatography (gel filtration), adsorption chromatography, ion-exchange chromatography, and affinity chromatography, HPLC, or a combination of these methods may be used, but is not limited thereto, and the desired product of L-arginine can be recovered from the medium or the microorganism using a suitable method known in the pertinent art.

Furthermore, the method for producing L-arginine of the present disclosure may additionally comprise a purification step. The purification can be performed using a suitable method known in the pertinent art. In one example, when the method for producing L-arginine of the present disclosure comprises both a recovery step and a purification step, the recovery step and the purification step may be performed continuously or discontinuously regardless of the order, or may be performed simultaneously or integrated into a single step, but is not limited thereto.

In the method of the present disclosure, variant, polynucleotide, vector, and strain, etc., are as described in the other aspects above.

Another aspect of the present disclosure is to provide a composition for producing L-arginine comprising at least one selected from the group consisting of the protein variant of the present disclosure, a polynucleotide encoding the protein variant, and a vector comprising the polynucleotide, a microorganism of the genus *Corynebacterium* comprising the same; a medium in which the same is cultured; or a combination of two or more of these.

The composition of the present disclosure may further include any suitable excipient commonly used in a composition for producing amino acids (e.g., L-arginine), and such an excipient may be, for example, a preservative, a wetting agent, a dispersing agent, a suspending agent, a buffering agent, a stabilizer, or an isotonic agent, but is not limited thereto.

In the composition of the present disclosure, variant, polynucleotide, vector, strain, medium, and L-arginine, etc., are as described in the other aspects above.

According to another aspect of the present disclosure, the present disclosure can provide a method for increasing L-arginine production ability of the microorganism, a method for imparting L-arginine production ability to the microorganism, or a method for preparing a microorganism with increased L-arginine production ability, comprising a step of introducing (for example, transformation) the above-described novel protein variant of the present disclosure, a polynucleotide encoding the variant, and/or a recombinant vector comprising the polynucleotide into the microorganism.

In the method for preparing a microorganism of the present disclosure, the polynucleotide, recombinant vector, and microorganism, etc., are as described above.

According to another aspect of the present disclosure, the present disclosure provides one or more uses selected from the group consisting of the protein variant of the present disclosure described above; a polynucleotide encoding the protein variant; a recombinant vector comprising the polynucleotide; and a microorganism comprising the protein variant, a polynucleotide encoding the protein variant, and/or a recombinant vector comprising the polynucleotide, for producing L-arginine, and/or producing an L-arginine-producing microorganism, and/or imparting and/or increasing the L-arginine production ability of a microorganism.

In the use for producing L-arginine, and/or producing an L-arginine-producing microorganism, and/or imparting and/or increasing the L-arginine production ability of a microorganism, the protein variant, polynucleotide, recombinant vector, and microorganism, etc., are as described above.

According to other aspects of the present disclosure, the present disclosure provides a composition, method, product, process, or use characterized by one or more elements disclosed in the present disclosure.

### [ADVANTAGEOUS EFFECTS]

When culturing a microorganism of the genus *Corynebacterium* comprising the novel protein variant of the present disclosure, it is possible to produce L-arginine in high yield compared to a microorganism having an existing non-modified polypeptide.

### [MODE FOR INVENTION]

Hereinafter, the present disclosure will be described in more detail through examples. These examples are only intended to describe the present disclosure in more detail, and it will be obvious to those of ordinary skill in the art that the scope of the present disclosure is not limited by these examples according to the gist of the present disclosure.

### EXAMPLE

(Throughout the present disclosure, "%" used to indicate the concentration of a specific substance is, unless otherwise specified, (weight/weight) % for solid/solid, (weight/volume) % for solid/liquid, and (volume/volume) % for liquid/liquid.)

### EXAMPLE 1. Construction of an L-arginine-producing microorganisms

### EXAMPLE 1-1. Construction of Corynebacterium glutamicum CJR2 strain

To evaluate L-arginine production capacity, *Corynebacterium glutamicum* CJR2, in which *ΔargR* and *argB(M54V)* mutations were introduced into wild-type *Corynebacterium glutamicum* ATCC13869, was prepared (Ikeda, Masato et al., Applied and environmental microbiology 75(6)1635-41, 2009).

First, vectors for introducing the *argR* deletion and the *argB(M54V)* mutation were prepared. PCR using primer pairs of SEQ ID NOs: 1 and 2, and SEQ ID NOs: 3 and 4, and overlapping PCR using the primer pair of SEQ ID NOs: 1 and 4 were performed using the genomic DNA of *Corynebacterium glutamicum* ATCC13869 as a template to obtain a homologous recombination fragment having the *argR* deletion mutation sequence. To prepare a homologous recombination fragment having the *argB*(*M54V*) mutation in the same manner, PCR using primer pairs of SEQ ID NOs: 5 and 6, and SEQ ID NOs: 7 and 8, and overlapping PCR using SEQ ID NOs: 5 and 8 were performed. The PCR reaction was performed by denaturation at 95°C for 5 minutes; repeating denaturation at 95°C for 30 seconds, annealing at 60°C for 30 seconds, and polymerization at 72°C for 1 minute 27 times; followed by a polymerization reaction at 72°C for 5 minutes. After the fragments obtained above purification process, fusion cloning was performed using an In-Fusion^{®} HD cloning kit (Clontech) by cloning with the pDC24 vector (SEQ ID NO: 81) treated with SmaI restriction enzyme according to the manual, to obtain plasmids. The prepared vectors were named pDC24-ΔargR and pDC24-argB(M54V), respectively.

Subsequently, the *argR* deletion mutation was introduced into wild-type *Corynebacterium glutamicum* ATCC13869. Transformation was performed by electroporation using the prepared pDC24-AargR plasmid (van der Rest et al., Appl Microbiol Biotechnol 52:541-545, 1999). Then, secondary recombination was performed on a solid plate medium containing 4% sucrose, and PCR using the primer pair of SEQ ID NOs: 1 and 4 was performed on the transformants in which the secondary recombination was completed to confirm that the deletion mutation was introduced into the argR gene on the chromosome. At this time, the PCR reaction was performed under the same conditions as described above, and the transformant thus obtained was named CJR1.

### <Solid plate medium (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Agar 20 g (based on 1 liter of distilled water)
The *argB(M54V)* mutation was introduced into the *Corynebacterium glutamicum* CJR1 in the same manner as above. The prepared pDC24-argB(M54V) plasmid was used, and PCR using the primer pair of SEQ ID NOs: 5 and 8 was performed on the transformants in which the secondary recombination was completed to confirm that the M54V mutation was introduced into the *argB* gene on the chromosome, and the transformant was named CJR2.

The sequence information of the primers used in Example 1-1 is described in Table 2 below.

**[Table 2]**

| SEQ ID NO | Name | SEQUENCE(5'->3') |
|---|---|---|
| 1 | *argR-5'-F* | tgaattcgagctcggtaccccactggtgaactccttgtcc |
| 2 | *argR-5'-R* | ttgaactaggggcgctttaaaagttttccggtgttgacgg |
| 3 | *argR-3'-F* | ccgtcaacaccggaaaacttttaaagcgcccctagttcaa |
| 4 | *argR-3'-R* | gtcgactctagaggatcccccgttgaactgcttgccagcc |
| 5 | *argB-5'-F* | tgaattcgagctcggtaccctgcggctcgcacggttgctc |
| 6 | *argB-5'-R* | acggtgcgcaagaagaccacgtcggcagcaaaagcagcct |
| 7 | *argB-3'-F* | ggctgcttttgctgccgacgtggtcttcttgcgcaccgtg |
| 8 | *argB-3'-R* | gtcgactctagaggatccccctcttatcaggccaatcggt |

### EXAMPLE 1-2. Construction of Corynebacterium glutamicum CJR100 strain

Based on the CJR2 strain prepared in Example 1-1, a CJR100 strain in which the enhanced N-acetyl-gamma-glutamyl-phosphate reductase (hereinafter, *argC*) gene was prepared.

In order to enhance the activity of *argC* (NCBI Accession No. BBD29_RS07530), which is an N-acetyl-gamma-glutamyl-phosphate reductase, a plasmid for enhancing *argC* activity was prepared by replacing the wild-type promoter of the *argC* gene with Po2 using the Po2 promoter (US 10273491 B2) known as a strong promoter. Upstream and downstream regions of the *argC* gene were obtained. Specifically, to prepare a strain into which *argC* with the Po2 promoter was introduced, PCR was performed to amplify the upstream region of the *argC* gene using primers of SEQ ID NO: 9 and SEQ ID NO: 10 and the gene fragment of the downstream region of the *argC* gene using primers of SEQ ID NO: 11 and SEQ ID NO: 12, respectively, using the chromosomal DNA of *Corynebacterium glutamicum* ATCC13869 as a template. In addition, a Po2 promoter fragment was obtained using SEQ ID NO: 13 and SEQ ID NO: 14 with the synthesized Po2 promoter as a template. As a polymerase for the PCR reaction, Pfu UltraTM High-Fidelity DNA Polymerase (Stratagene) was used, and PCR was performed in the same manner as in Example 1-1. As a result, an 86bp DNA fragment of the Po2 promoter region, a 610bp DNA fragment of the *Corynebacterium glutamicum* ATCC13869 *argC* upstream, and a 1086bp DNA fragment of the downstream were obtained, respectively. PCR was performed in the same manner as in Example 1-1 using the amplified promoter and DNA fragments as templates and using the primers of SEQ ID NO: 9 and SEQ ID NO: 12. After the two fragments obtained above were subjected to DNA purification, fusion cloning was performed by ligating them with a pDC24 plasmid treated with SmaI restriction enzyme using an In-Fusion^{®} HD cloning kit (Clontech). The resulting vector was named pDC24-Po2-argC.

Subsequently, the CJR2 strain prepared in Example 1-1 was transformed with the prepared pDC24-Po2-argC plasmid by electroporation (van der Rest et al., Appl Microbiol Biotechnol 52:541-545, 1999). Then, secondary recombination was performed on a solid plate medium containing 4% sucrose, and PCR was performed on the transformants for which the secondary recombination was completed using primers of SEQ ID NOs: 9 and 14 to confirm that the chromosomal *argC* gene was enhanced with the Po2 promoter. At this time, the PCR reaction was performed under the same conditions as described above, and the transformant thus obtained was named CJR100.

### <Solid plate medium (pH 7.0)>

Glucose 10 g, Peptone 10 g of, beef extract 5 g, yeast extract 5 g, Brain Heart Infusion 18.5 g, NaCl 2.5 g, urea 2 g, sorbitol 91 g, and agar 20 g(based on 1 liter of distilled water)

The sequence information of the primers used in Example 1-2 is described in Table 3 below.

**[Table 3]**

| SEQ ID NO | Name | SEQUENCE(5'->3') |
|---|---|---|
| 9 | *argC-5'-F* | GTGAATTCGAGCTCGGTACCCGCCCCGAAAAGCCGTT AAAAG |
| 10 | *argC-5'-R* | tgccaaaattcacgattattgCTCGAGTCTAGAGACGGGTTA |
| 11 | *argC-3'-F* | ttattggaggagatcaaaacaATGACAATCAAGGTTGCAATC |
| 12 | *argC-3'-R* | CAGGTCGGCGTCGCACCTTAAGGGGATCCTCTAGAGT CGACC |
| 13 | Po2-F | caataatcgtgaattttggca |
| 14 | Po2-R | tgttttgatctcctccaataa |

### EXAMPLE 2: Selection of mutant strain with increased arginine production ability by artificial mutagenesis

### EXAMPLE 2-1: Random mutagenesis via UV irradiation

To select a mutant strain with increased arginine production ability, the arginine-producing strain of CJR100 prepared in Example 2-1, was spread on a nutrient medium containing agar and cultured at 30°C for 16 hours. The hundreds of colonies thus obtained were irradiated with UV (Ultraviolet mutation) at room temperature to induce random mutations in the genome of the strain.

### <Nutrient Medium (pH 7.2)>

Glucose 10 g, meat extract 5 g, polypeptone 10 g, sodium chloride 2.5 g, yeast extract 5 g, agar 20 g, urea 2 g (per 1 liter of distilled water)

### EXAMPLE 2-2: Selection of strain with increased L-arginine production ability

To select a mutant strain with increased arginine productivity compared to the parent strain CJR100, CJR100 strain and the mutant strains in which random mutations were induced in Example 2-1, were cultured by the following method.

Each of the aforementioned strains was inoculated into a 96-Deep Well Plate-Dome (Bioneer) containing 400 µl of a seed medium, and cultured in a plate shaking incubator (TAITEC) at 32°C and 1200 rpm for about 48 hours. The arginine concentrations of about 3000 cultured strains were respectively checked via a near-infrared (NIR) spectroscopic analyzer, and the top 4 mutant strains with improved arginine productivity compared to the parent strain CJR100 were selected.

### <Seed Medium (pH 7.0)>

Glucose 20 g, Peptone 10 g, yeast extract 5 g, urea 1.5 g, KH₂PO₄ 4 g, K₂HPO₄ 8g, MgSO₄·7H₂O 0.5 g, biotin 100 µg, thiamine HCl 1000 µg, calcium pantothenate 2000 µg, nicotinamide 2000 µg (based on 1 liter of distilled water)

To finally select strains with reproducibly increased L-arginine production ability from the four selected mutant strains, they were cultured and evaluated for produced L-arginine concentration by the following method.

The control stain and the aforementioned four mutant strains were inoculated respectively into a 250 mL corner-baffle flask containing 25 mL of an arginine production medium, and then cultured with shaking at 32°C for 20 hours at 200 rpm. Then, 1 ml of a seed culture was inoculated into a 250 ml corner-baffled flask containing 24 ml of a production medium, and shake-cultured at 30°C and 200 rpm for 54 hours.

### <Seed Medium (pH 7.0)>

Glucose 20 g, Peptone 10 g, yeast extract 5 g, urea 1.5 g, KH₂PO₄ 4 g, K₂HPO₄ 8 g, MgSO₄·7H₂O 0.5 g, biotin 100 ug, thiamine HCl 1000 ug, calcium-pantothenate 2000 ug, nicotinamide 2000 ug (based on 1 liter of distilled water)

### <Production medium (pH 7.2)>

5% glucose, 3% ammonium sulfate, 0.1% potassium phosphate monobasic, 0.2% magnesium sulfate heptahydrate, 1.5% CSL (corn steep liquor), 1% NaCl, 0.5% yeast extract, biotin 100 mg/L
After the cultivation was completed, the L-arginine concentration in the culture broth was analyzed using high-performance liquid chromatography (HPLC), and the L-arginine production concentrations of each mutant strain are shown in Table 4 below.

**[Table 4]**

| Strain name | L-Arg (g/L) |
|---|---|
| CJR100 | 5.8 |
| CJR100_mt1 | 6.0 |
| CJR100_mt2 | 2.5 |
| CJR100_mt3 | 6.3 |
| CJR100_mt4 | 5.9 |

As shown in Table 4 above, among the four selected mutant strains, CJR100_mt3 was finally selected as the mutant strain with the greatest increase in L-arginine production.

### Example 3. Identification of mutations through whole-genome sequencing (WGS)

A gene having a change in protein sequence due to a nucleotide sequence mutation was identified by performing whole-genome sequencing (WGS) on the CJR100_mt3 strain selected in Example 2-2 above and comparing it with the parent strain CJR100, and the amino acid sequence of the protein variant and the nucleotide sequence of the gene are described in Table 5 and Table 6 below.

**[Table 5]**

| Mutation of CDS (Coding Sequence) region | | | | | | | |
|---|---|---|---|---|---|---|---|
| Amino acid of wild-type strain | Position of mutation for protein | Amino acid of mutant strain | gene ID | Gene Name | protein | SEQ ID NO of mutant amino acid sequence | SEQ ID NO of mutant nucleotide sequence |
| A | 306 | S | BBD29_0 0030 | - | transposase | 15 | 21 |
| P | 65 | L | BBD29_0 5930 | - | HNH nuclease | 16 | 22 |
| A | 365 | Deletion* | BBD29_0 8260 | - | Cell wall-associated hydrolase | 17 | 23 |
| V | 123 | A | BBD29 _0 2985 | tetR2 | TetR family transcripti onal regulator | 18 | 24 |
| P | 432 | L | BBD29-0 6380 | - | transposase | 19 | 25 |
| G | 922 | E | BBD29_0 7445 | uvrA | excinuclease ABC subunit A | 20 | 26 |

| | | | | | | | |
|---|---|---|---|---|---|---|---|
| * Deletion: By a nucleotide sequence substitution of a gene, a codon encoding an original amino acid is mutated into a stop codon, so as to delete an amino acid sequence at the corresponding position. | | | | | | | |

**[Table6]**

| Mutation of RNA region | | | | | | |
|---|---|---|---|---|---|---|
| **RNA TYPE** | **Name** | **Gene name** | **Expressed protein** | **Mutation type** | **SEQ ID NO of CJR100 nucleotide sequence** | **SEQ ID NO of nucleotide sequence** |
| rRNA | BBD29 _12405 | rRNA01 | 23S ribosomal RNA | substitution | 27 | 30 |
| rRNA | BBD29 _07610 | rRNA04 | 23S ribosomal RNA | Substitution & insertion | 28 | 31 |
| rRNA | BBD29 _04520 | rRNA05 | 23S ribosomal RNA | insertion | 29 | 32 |

**[Table 7]**

| SEQ ID NO | Sequence (N-terminus→ C-terminus for amino acid sequence; 5'→3' for nucleotide sequence) |
|---|---|
| 15 | MPKPLPPETRRKIIDFDPFAPNSPSIEEFCSRLKISRRSFYNIRNRYQQDANA ALHPRSSAPITARRTYDESITSTLLSIRARLKAQGWEYGPISIRFEGISTGEL TAPIPSVSTIARLLRAAGAVESNPKKRPKSSVVRFQRGQAMEMWQIDGFI YTLHDTDLTRVTIYQILDDATRFDVGTCVFPANENSVDARTVLEQAIAHFG APHELLSDNGSAFNRMRQGYVGSLESYLATVGCLSITGKPGHPQTQGKN ERSHRTLFRFLQAHQPHTLEECAHYIEQFRDHYNNRRPHQGLPNNLTPAS AWEIVGCVEQQPPIDPVVLQQQADHYARRRIEKQSDVPLVRQEESVPVSK MQQTEQRRRVRRFGKKPVDPDVFTFAGNNQKVVFQGMRISVPRTMRDR EFYRTVTATELGFWDAITGELELSIPLLVVAIARGKSYVNSYNIRGVWMQ HPTPLWQRKRDEAEKRFKSIDPGDLLR |
| 16 | MTTDIYFSHNNPHDPYAHHTTELNRDTHHLWVTLTTDSDDFDADSFTTEV IRITGYSRHEVNNG |
| 17 | VANLDSVRPGVRAALRRTHTQVKGVALTRALIALAVSGALLSSMTPAVAQ PQNPDDAAIAQAEENVSAGDGEVARLAGSLSSTDAEINRVELEMGALREE VNKSLVDLHDAQAIAEQARQDALAAKKDLDDSQAQIEAAQERLDEISRA AYRQNGTSKGLSGISGNGNSEDALDRQTYLRTSAEKQQAAVEELDRLRTE NANKESVLRQARIVAEQREAEAVEKQVQTEAAIAANSEQLNVLTNNRTTL VAQRDEAERNLAIARAQADNLQGQRAEYEEFQQAEQARIQAEAEAQAA AEEKRRADEAAAQAAAEAQEAAQQAQAAEEAQAAQAAEAAQAQATQA AETQAAETQAAETQAAETQA |
| 18 | MTQSLRDAKRESTRRALAETTYRMVRARGFDQVTIDEITTEVNVSRRTFS NYFANKEEAVAFVPVDRIRTELEKWEAPAGIDAAAAVRSVVAHLLASGLP MMLAELGRLGRRHPPFGPHSREAQWAVWDAVRERLHEVLHIDAPRDRR NLAMLLGALYGIVSTQLADLDLGDEGPGPEVFLALQESVGEVFTLFEPAL RRIGQ |
| 19 | MPKPLPPETRRKIIDFDPFAPNSPSIEEFCSRLKISRRSFYNIRNRYQQDANA ALHPRSSAPITARRTYDESITSTLLSIRARLKAQGWEYGPISIRFEGISTGEL TAPIPSVSTIARLLRAAGAVESNPKKRPKSSVVRFQRGQAMEMWQIDGFI YTLHDTDLTRVTIYQILDDATRFDVGTCVFPANENSVDARTVLEQAIAHFG APHELLSDNGSAFNRMRQGYVGSLESYLATVGCLSITGKPGHPQTQGKN ERSHRTLFRFLQAHQPHTLEECAHYIEQFRDHYNNRRPHQGLPNNLTPAA AWEIVGCVEQQPPIDPVVLQQQADHYARRRIEKQSDVPLVRQEESVPVSK MQQTEQRRRVRRFGKKPVDPDVFTFAGNNQKVVFQGMRISVPRTMRDR EFYRTVTATELGFWDAITGELELSIPLLVVAIARGKSYVNSYNIRGVWMQ HPTPLWQRKRDEAEKRFKSIDPGDLLR |
| 20 | MADRLVVRGAREHNLKGVDIDLPRDSMVVFTGLSGSGKSSLAFDTIFAEG QRRYVESLSSYARMFLGQMDKPDVDLIDGLSPAVSIDQKSTNRNPRSTVG TITEVYDYLRLLYARAGTAHCPVCDARVERQTPQQMVDQILGMEEGLKF QILAPVVRTRKGEFVDLFADLASQGYSRVRVDGEVHQLSDPPKLEKQIKH DIDVVVDRLQVKASQKQRLTDSMETALRLADGVAVLEFVGLEEDDPNRL RRFSEKMSCPNGHALTVDELEPRAFSFNSPYGACPACDGLGVRTEVDIDLI IPDPDAPATKAVQPWNSSPNHSYFEKLIEGLAKALGFDPETPYSELTAAQK KALVYGSKEEVSVRYKNRYGRVRSWTAPFEGVMGYFDRKLEQTDSETQ KDRLLGYTREVPCPTCKGARLKPEILAVRLDSGSHGALSIAGLTALSVHEA FEFLDNLTLGKREEMIAGAVLREIHARLKFLLDVGLSYLTLDRAAGTLSG GEAQRIRLATQIGSGLAGVLYVLDEPSIGLHQRDNQRLITTLEHLRDIGNT LIVVEHDEDTIRRADWLVDIGPRAGEFGGEVVYQGEPKGILDCEESLTGA YLSGRRTLGVPDTRREIDKERQLKVVGARENNLRGIDVKIPLGVLCCITG VSGSGKSTLVNQILAKVLANKLNRARQVPGRAKRVEGLEHLDKLVQVDQ SPIGRTPRSNPATYTGVFDKVRNLFAETTEAKVRGYKPGRFSFNIKGGRCE ACQGDGTLKIEMNFLPDVYVPCEVCDGQRYNRETLEVKYKGKNIAEVLG MPISEAADFFEPITSIHRYLATLVDVGLGYVRLGQAATTLSGGEAQRVKLA AELQKRSNGRTVYILDEPTTGLHFEDIRKLMMVIQGLVDKGNSVIIIEHNL DVIKAADWIVDMGPEGGSGGETVVAEGTPEQVAEVAGSYTGQFLKELL |
| 21 | ATGCCTAAACCCCTACCCCCAGAAACCCGACGCAAGATCATCGATTTC GATCCGTTCGCACCGAACAGCCCCTCGATCGAAGAGTTCTGCAGTCGG CTAAAAATATCGCGGCGCAGCTTCTACAACATCCGCAACCGATACCAA CAAGACGCCAACGCAGCACTGCATCCACGCTCCAGCGCCCCAATCACC GCCCGGCGAACGTACGATGAATCCATCACCAGCACCTTGCTGTCCATC CGCGCACGCCTGAAAGCCCAAGGATGGGAGTACGGCCCGATCTCTATT CGATTCGAAGGCATCTCCACCGGGGAACTGACTGCACCGATTCCATCT GTTTCAACCATTGCTCGTTTGTTACGCGCCGCAGGAGCTGTTGAAAGT AACCCTAAGAAGCGACCGAAGTCGTCGGTGGTGAGGTTTCAACGAGG TCAAGCCATGGAAATGTGGCAAATAGATGGTTTCATCTACACACTGCAC GATACTGATCTGACTCGGGTAACGATCTATCAAATCCTTGATGATGCCA CTCGGTTTGATGTCGGAACCTGTGTCTTTCCCGCGAATGAAAACTCAG TCGATGCCCGAACAGTGCTAGAACAAGCCATTGCTCATTTTGGTGCCC CGCATGAATTACTCAGTGATAACGGCTCAGCGTTTAACCGGATGCGGC AAGGATACGTCGGAAGCTTGGAAAGCTATCTCGCGACGGTGGGGTGTT TAAGCATTACCGGCAAGCCTGGGCATCCTCAAACCCAAGGTAAAAACG AACGCAGCCACCGCACACTATTTCGTTTCCTTCAAGCTCATCAACCAC ACACACTTGAGGAATGCGCGCACTATATTGAACAGTTCCGCGATCATTA CAACAATCGTCGCCCGCATCAGGGGTTGCCGAATAATCTCACCCCGGC CTCGGCCTGGGAGATTGTGGGCTGTGTGGAACAACAACCCCCGATTGA TCCCGTCGTGCTTCAACAGCAAGCCGATCACTATGCCCGTCGTCGAATT GAGAAGCAATCAGATGTCCCGCTTGTTCGGCAGGAGGAATCTGTTCCG GTGTCTAAGATGCAGCAGACCGAGCAACGTCGACGGGTACGTCGTTTT GGTAAAAAGCCGGTGGATCCTGATGTGTTTACGTTTGCTGGGAATAATC AGAAGGTGGTGTTCCAGGGGATGCGTATTTCTGTTCCTCGTACGATGAG GGACAGGGAGTTTTATCGGACGGTGACGGCAACTGAGCTTGGTTTCTG GGATGCGATAACCGGGGAGCTTGAGTTGTCGATTCCGTTGCTGGTTGT GGCGATTGCCCGGGGTAAGTCGTATGTCAATTCTTACAACATTCGTGGG GTGTGGATGCAACACCCGACACCGTTGTGGCAACGCAAACGTGATGA GGCTGAGAAGCGATTTAAGTCGATAGATCCGGGGGATCTTCTGCGCTA G |
| 22 | ATGACCACTGACATCTACTTCAGCCACAACAACCCACACGACCCATAC GCCCACCACACCACCGAACTCAACCGCGACACCCATCACCTCTGGGTC ACCCTCACCACCGACTCCGACGACTTCGACGCAGACTCCTTCACCACC GAAGTCATCCGGATCACCGGCTACTCCCGCCACGAAGTCAACAACGGC CTTAACGCCATGGCCGCGATGACCAACCTCCCACACCTACGCGCCATC CAAGAACGCTACTACTTCCTGAGCATCCGCTACCTCGCCTCCATCATGA TCGCCGTGGCCAAAGCAGACCCCACCCTGTGGGAAGAACTCGACCTG CGCATCACCGACGCCTTAACACCAGTCACCGCAGGGGAAGTCATGATC CAATCCTCCACCCTGGCTAAACGCATCGCCGCCTGGATCAAAGAACTC GACCCCGAACCCACCGAGGACCCCACACCGAAAGAGGACTATGTTCA CGTCCACACCACCGATGAGGCGACCTATGTCCGCATCAAAATCAGCGG CCCCAACCGCCTGCTCCTCAATGACATCATCACCCAACTCAAAGACAC AGACACCGAGGACAGCCTGCCTGAAGCGCTCATGGCCTTCCTGACAG AGAAAATCCAGTTAAAGATCACCAAATACCTCTTCACCCCACACAATC ACCCTGAGCAGGCATGGTCACCAGACTACGGTGACATTGATCCCGAAG CCTACGACAATGCCACCCTCGTGTGCGCCAAGGACTTAGATGAGGTCG CTGGAGCCACGGAGAAGAGCTACACCCCGAGTGAGAAGATGAAAGCC CTGATCAGAGCTCGGGATGGGCATTGCCGCTTCCCAGGGTGTTGCGTC CCGGCCAGTAAATGCCAGGTCGATCACATTATCCCGTGGGCGGAGGGC GGCCCGACAGCGGCGTGGAACCTGCAGTTGTTGTGCCAGCGGCATCA CAATATGAAAACCGATGGTCGCTTTACTGCTGATGCTAATGGATTGGCG GAGATTAGATGGATTGGGCCGATGGATGTACCAGCGGTAACTAGGCCG ACGGGTCCGTTGGTGAAAGCGATGCCGCGGGGGATTTGGGGTCAGGT GTTGAGGGATCGGATCCAGGCTAGGTTTGAGCGGATCCGCGACCGCGC CCTCAACAAAGAAGACTAG |
| 23 | GTGGCCAATCTAGATTCAGTTCGCCCTGGGGTGCGGGCAGCTCTTCGA CGTACCCATACCCAGGTAAAAGGTGTCGCGTTGACCAGGGCATTGATT GCGCTTGCAGTAAGCGGAGCTTTGCTTAGTTCCATGACTCCGGCGGTG GCGCAGCCACAGAATCCGGATGACGCAGCCATTGCACAGGCAGAGGA AAATGTTTCGGCGGGCGATGGGGAAGTCGCCCGCCTGGCAGGATCTTT GTCCAGCACTGACGCGGAAATTAACCGCGTCGAGCTGGAAATGGGTG CTCTGCGTGAAGAAGTGAACAAGTCCCTCGTGGATTTGCATGATGCGC AGGCAATCGCCGAACAGGCCCGCCAAGATGCTCTTGCAGCCAAGAAG GATCTCGATGATTCTCAAGCGCAGATCGAAGCAGCCCAAGAGCGCCTT GATGAGATTTCACGTGCAGCGTATCGCCAAAACGGAACCTCCAAGGGG CTTTCCGGCATTTCGGGCAATGGAAATTCTGAAGATGCGCTAGATCGTC AGACTTACCTGCGAACCAGTGCGGAAAAGCAGCAGGCAGCTGTTGAA GAGCTTGATCGCCTCCGTACGGAAAACGCCAACAAGGAATCGGTGTTG CGCCAGGCCCGCATCGTTGCTGAGCAGCGTGAGGCGGAAGCCGTCGA AAAGCAAGTCCAGACCGAGGCTGCAATTGCCGCAAACAGCGAGCAGC TCAATGTCTTGACTAACAACCGCACTACCTTGGTTGCCCAGCGTGATGA GGCTGAGCGCAACTTGGCCATCGCTCGTGCGCAGGCGGATAATCTGCA AGGTCAGCGTGCTGAGTACGAGGAATTCCAGCAGGCAGAGCAGGCTC GCATCCAGGCGGAAGCGGAAGCTCAGGCTGCTGCGGAGGAGAAGCGT CGTGCCGATGAGGCTGCTGCACAGGCAGCCGCTGAAGCTCAAGAGGC TGCCCAGCAAGCTCAGGCAGCGGAGGAAGCCCAAGCTGCGCAAGCAG CTGAGGCAGCACAGGCACAAGCAACTCAAGCTGCAGAAACCCAAGCT GCAGAAACCCAAGCTGCAGAAACCCAAGCTGCAGAAACCCAAGCTGC AGAAACCCAAGCTGCAGAAACCCAAGCTGCACAAGCCGCGCAAGCTC AGGCAGAAGCGAATGATCGTGCCGCCGCGCAACAGCGTGCTGCAGAG GCTCAAGCAGCAGCGGAACAGGCGCAACGTGAGGCTGACGCTCAGGC GGCCAACGATGCCCAAGCTCAGGTACTGCGTGAACAGGCGCTCACTG CAGCCTCCATCGCTGCGGCTGCTCTAATTGCGGCGAGCCAGTCCAGCC ATGCCACTACTCAAAATCCTTACCCAACTGATGAAGACGCGGATCCGA CCGATATTGCGGACATCCAAGGCCCAACGCAGCCAGGTACGGGTGAGT CTGGAGATTCCCAGAACGACTCCAGCGACAATGATTCCACAGGCAACG ATTCCACAGGCTCTGACTCTTCAGATTCAGATTCCTCCGGCAACGATTC TTCAGAGGTTATTTCCGGCGATCGTTCCGCTCAGATTGAGACTGTGATT GCGCGCGCCATGAGCCAGTTGGGTGTGCAGTACGCATGGGGTGGCGGT AACGCTAATGGCCCAACTCTGGGTATCCGTGACGGTGGCGTGGCGGAC TCTTACGGCGATTACAACAAGGTTGGCTTCGACTGCTCTGGACTGACC TTGTATGCGTTTGCGGGTGTGGGAATTTCACTTCCTCACTACACGGGCT ACCAGTACCAGCACGGCACCAAGGTGTCGCCTTCTGAGATGCAACGTG GCGATCTGATCTTCTATGGTCCGGGAGCGTCTCAGCACGTGGCAATTTA CCTCGGTGATGGTCAGATGATTGAGGCTCCGAATTCGGGTTCTGTCGTG AAGATTTCTCCTGTTCGCTGGAGCGGAATGACCGAGAGCGTGGTACGC CTCATTTAG |
| 24 | ATGACGCAGAGCCTGAGAGACGCCAAGCGGGAGTCGACCCGGCGAGC GCTCGCCGAGACCACCTACCGGATGGTGCGTGCCCGCGGTTTCGACCA GGTGACCATCGACGAGATCACCACCGAGGTCAACGTCTCCCGCCGTAC CTTCTCCAATTACTTCGCCAACAAAGAGGAGGCGGTCGCCTTCGTGCC GGTCGACCGCATCCGGACGGAACTGGAGAAGTGGGAGGCGCCGGCGG GCATCGACGCGGCGGCTGCCGTGCGTTCCGTCGTCGCCCACCTGCTCG CCAGCGGGCTGCCGATGATGCTCGCCGAGCTCGGTCGCCTGGGCCGCC GTCACCCCCCGTTCGGCCCCCACAGCAGGGAGGCCCAGTGGGCGGTG TGGGACGCGGTCCGGGAAAGGCTCCACGAGGTGCTGCACATCGATGC CCCCCGTGACCGCCGTAACCTCGCGATGCTGCTCGGCGCGCTCTACGG CATCGTGTCGACGCAGCTCGCTGACCTGGATCTGGGCGACGAGGGGCC GGGCCCCGAGGTGTTCCTGGCGCTGCAGGAGAGCGTGGGGGAGGTGT TCACGCTGTTCGAACCGGCCCTGCGCCGGATCGGGCAATGA |
| 25 | ATGCCTAAACCCCTACCCCCAGAAACCCGACGCAAGATCATCGATTTC GATCCGTTCGCACCGAACAGCCCCTCGATCGAAGAGTTCTGCAGTCGG CTAAAAATATCGCGGCGCAGCTTCTACAACATCCGCAACCGATACCAA CAAGACGCCAACGCAGCACTGCATCCACGCTCCAGCGCCCCGATCACC GCCCGGCGAACGTACGATGAATCCATCACCAGCACCTTGCTGTCCATC CGCGCACGCCTGAAAGCCCAAGGATGGGAGTACGGCCCGATCTCTATT CGATTCGAAGGCATCTCCACCGGGGAACTGACTGCACCGATTCCATCT GTTTCAACCATTGCTCGTTTGTTACGCGCCGCAGGAGCTGTTGAAAGT AACCCTAAGAAGCGACCGAAGTCGTCGGTGGTGAGGTTTCAACGAGG TCAAGCCATGGAAATGTGGCAAATAGATGGTTTCATCTACACACTGCAC GATACTGATCTGACTCGGGTCACGATCTATCAAATCCTTGATGATGCCA CTCGGTTTGATGTCGGAACCTGTGTCTTTCCCGCGAATGAAAACTCAG TCGATGCCCGAACAGTGCTAGAACAAGCCATTGCTCATTTCGGTGCCC CGCATGAATTACTCAGTGATAACGGCTCAGCGTTTAACCGGATGCGGC AAGGATACGTCGGAAGCTTGGAAAGCTATCTCGCGACGGTGGGGTGTT TAAGCATTACCGGCAAGCCTGGGCATCCTCAAACCCAAGGTAAAAACG AACGCAGCCACCGCACACTATTTCGTTTCCTTCAAGCTCATCAACCAC ACACACTTGAGGAATGTGCGCACTATATTGAACAGTTCCGCGATCATTA CAACAATCGTCGCCCGCATCAGGGGTTGCCGAATAATCTCACCCCGGC CGCGGCCTGGGAGATTGTGGGCTGTGTGGAACAACAACCCCCGATTGA TCCCGTCGTGCTTCAACAGCAAGCCGATCACTATGCCCGTCGTCGAATT GAGAAGCAATCAGATGTCCCGCTTGTTCGGCAGGAGGAATCTGTTCCG GTGTCTAAGATGCAGCAGACCGAGCAACGTCGACGGGTACGTCGTTTT GGTAAAAAGCCGGTGGATCCTGATGTGTTTACGTTTGCTGGGAATAATC AGAAGGTGGTGTTCCAGGGGATGCGTATTTCTGTTCCTCGTACGATGAG GGACAGGGAGTTTTATCGGACGGTGACGGCAACTGAGCTTGGTTTCTG GGATGCGATAACCGGGGAGCTTGAGTTGTCGATTCCGTTGCTGGTTGT GGCGATTGCCCGGGGTAAGTCGTATGTCAATTCTTACAACATTCGTGGG GTGTGGATGCAACACCCGACACCGTTGTGGCAACGCAAACGTGATGA GGCTGAGAAGCGATTTAAGTCGATAGATCCGGGGGATCTTCTGCGCTA G |
| 26 | TTGGCTGATCGCCTCGTAGTGCGCGGAGCGCGTGAACACAACCTAAAA GGCGTGGATATTGATCTGCCACGCGACTCGATGGTGGTGTTCACCGGC CTGTCAGGTTCCGGTAAATCATCACTGGCCTTTGACACCATCTTTGCGG AAGGCCAGCGCCGTTACGTGGAGTCGTTGTCCAGTTACGCCCGCATGT TCTTGGGGCAGATGGACAAGCCGGACGTGGATTTGATTGATGGATTATC CCCAGCGGTCTCCATTGACCAAAAGTCCACCAACCGCAACCCTCGGTC CACCGTCGGTACCATCACGGAAGTCTATGACTACCTGCGTCTTCTGTAT GCCCGCGCTGGTACCGCACACTGCCCAGTGTGTGATGCCCGCGTGGAG CGTCAAACCCCGCAGCAGATGGTGGACCAGATCCTTGGCATGGAGGA GGGACTGAAGTTCCAAATCCTTGCGCCTGTGGTGCGTACCCGTAAAGG TGAGTTCGTTGATCTTTTCGCAGATCTTGCATCCCAAGGTTATTCCCGC GTGCGGGTTGATGGGGAAGTGCACCAGCTCTCGGATCCTCCAAAGCTA GAAAAGCAGATCAAGCACGATATTGATGTTGTGGTTGACCGTCTGCAG GTAAAAGCCAGCCAAAAGCAGCGCCTGACAGACTCTATGGAAACCGC ACTTCGCCTGGCCGATGGCGTGGCTGTGCTGGAGTTCGTTGGCCTGGA GGAAGATGATCCGAATAGGCTTCGTCGATTCTCTGAAAAGATGAGCTG CCCTAACGGTCACGCGTTGACGGTTGATGAGCTGGAGCCTCGTGCTTT TTCCTTCAACTCTCCTTATGGCGCGTGTCCTGCCTGTGATGGCTTGGGT GTGCGCACCGAAGTTGATATTGATCTGATCATCCCAGATCCAGATGCAC CTGCAACTAAAGCGGTTCAGCCCTGGAACTCCAGCCCAAACCACTCTT ACTTTGAAAAGCTCATTGAAGGCCTGGCGAAAGCCCTCGGATTTGATC CGGAAACTCCGTACAGTGAGCTCACCGCAGCTCAAAAGAAGGCTCTG GTCTATGGATCGAAGGAAGAAGTAAGCGTTCGATACAAGAACCGCTAC GGACGCGTGCGTTCTTGGACTGCGCCTTTTGAAGGTGTCATGGGCTAC TTTGATCGCAAGTTGGAGCAGACTGATTCCGAAACCCAAAAAGACCG ACTGTTGGGCTACACCCGTGAAGTGCCCTGCCCAACCTGTAAAGGCGC ACGCCTCAAGCCGGAAATCTTGGCCGTACGCCTAGACTCCGGAAGCCA TGGAGCGTTGTCCATTGCTGGACTAACCGCGCTGTCGGTGCATGAAGC ATTCGAGTTTTTGGATAACCTCACACTGGGCAAGCGCGAGGAAATGAT CGCGGGAGCTGTGCTGAGGGAAATTCACGCCCGCCTGAAATTCTTGCT TGACGTGGGCCTTTCCTACCTCACCCTTGATCGCGCCGCAGGCACCCT GTCTGGTGGTGAAGCGCAGCGTATCCGCCTGGCTACTCAAATTGGTTC CGGTCTGGCTGGTGTGCTCTACGTCTTGGATGAGCCATCCATTGGTCTG CACCAACGTGACAACCAGCGCTTAATCACTACCCTTGAGCATCTCCGA GATATCGGAAACACACTCATTGTTGTGGAACACGATGAAGACACCATC AGGCGCGCAGATTGGCTCGTGGATATTGGTCCTCGAGCTGGTGAATTT GGTGGTGAAGTGGTCTACCAAGGTGAGCCGAAGGGCATTTTGGACTG CGAAGAATCCCTCACAGGTGCTTACTTGTCTGGTCGTCGAACCCTGGG TGTTCCTGATACTCGCCGTGAGATCGACAAAGAGCGACAGCTCAAGGT GGTTGGTGCTAGGGAAAACAACCTGCGGGGCATCGATGTGAAAATCCC ACTGGGTGTGCTGTGCTGCATCACTGGTGTGTCGGGATCTGGTAAATCC ACGCTGGTCAATCAGATTTTGGCCAAGGTTCTGGCCAACAAACTCAAC CGCGCACGCCAAGTGCCTGGTCGCGCAAAGCGGGTGGAAGGCCTCGA GCACTTGGATAAGTTGGTCCAGGTGGATCAGTCGCCAATTGGTCGTACT CCACGTTCAAACCCAGCGACGTACACGGGTGTGTTTGATAAAGTCCGT AACCTTTTTGCCGAGACCACTGAAGCGAAGGTCCGCGGTTACAAGCCT GGCCGCTTCTCCTTCAATATTAAGGGTGGACGCTGCGAAGCATGTCAG GGCGATGGCACGCTGAAGATCGAAATGAACTTCCTGCCCGACGTGTAT GTTCCGTGTGAAGTCTGTGATGGTCAGCGCTACAACCGCGAGACCCTC GAGGTGAAGTACAAGGGCAAAAACATCGCTGAAGTATTGGGCATGCC GATCTCTGAGGCTGCGGACTTCTTTGAGCCCATCACCTCAATTCACCGA TACCTAGCAACGCTGGTTGATGTCGGCCTTGGCTATGTCCGTTTGGGCC AGGCAGCAACAACCTTGTCTGGTGGTGAAGCCCAGCGTGTGAAACTT GCCGCTGAGCTGCAGAAGCGTTCCAACGGTCGCACCGTTTACATCCTC GATGAGCCAACTACTGGTTTGCACTTTGAAGATATTCGCAAACTCATGA TGGTGATCCAAGGCCTGGTGGACAAGGGTAACTCCGTGATCATCATCG AGCACAACCTCGACGTGATCAAGGCTGCCGACTGGATCGTGGACATGG GTCCAGAAGGCGGAAGCGGCGGTGAAACTGTGGTCGCTGAAGGAACC CCAGAGCAAGTTGCTGAAGTTGCGGGTTCCTACACCGGCCAATTCCTT AAAGAGTTGTTGTAG |
| 27 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCACCCGTTTGTGGTGTGATGGCGTGCCTTTTGAAGAATGAGCCTGCG AGTCAGCGGCATGTCGCGAGGTTAACCCGTGTGGGGTAGTCGTAGGGA AACCGAATCCTAACGAGGGTGTTTTTAGTGGCATGTCTTGGACCCGAA GCGGAGTGATCTACCCATGGCCAGTGTGAAGCAGCTGTAAGAGGTTGT GGAGGCGCGAACCCACTTAGGTTGAAAACTGAGGGGATGAGTTGTGG GTAGGGGTGAAAGGCCAATCAAACTCCGTGATAGCTGGTTCTCCCCGA AATGCATTTAGGTGCAGCGTCGTGTGTTTCTTGCCGGAGGTAGAGCTAC TGGATGGTTTAGCGGGACCAACATCTTAGCGACATCAGCCAAACTCCG AATGCCGGTAAGTTAGAGCACGGCAGTGAGACTGCGGGGGATAAGCTT CGTAGTCGAGAGGGAAACAGCCCAGATCGCCGGCTAAGGCCCCTAAG GGTGTGCTAAGTGGAAAAGGAGGTGGGGTCGCGAAGACAGCCAGGA GGTTGGCTTAGAAGCAGCCATCCTTGAAAGAGTGCGTAATAGCTCACT GGTCGAGTGATTCCGCGCCGACAATGTAGTGGGGCTTAAGTACACCGC CGAAGCCGCGGCAATGATCTTTATAGGATTGTTGGGTAGGGGAGCGTC GTGCATGCGTTGAAGCTTTGGGGTGACCTTGGGTGGAGTGTGTGCGAG TGAGAATGCAGGCATGAGTAACGAATGATGCGTGAGAAACGTATCCGC CGGATGACTAAGGGTTCCTGGGTCAAGTTAATCTTCCCAGGGTGAGTC GGGGCCTAAGGCGAGGCCGACAGGCGTAGTCGATGGATAACGGGTTG ATATTCCCGTACCCGAGTATGAGCGACCATGGTGAATCAGTGATACTAA CCACCCATAAGCACCCGCGAAAAGGCTTTGCTTTTTTGTGGTGTGTGG TTGCGTGGGACCTGATCTGGTAGTAGCTAAGTGATGGGGTGACGCAGG GAGGTAGCTCAGCCACTTATTGGATTGTGGTGTAAGCGTGTGGCACGC AGTGTAGGTCAAATCCGCACTGTTTTTGTGTGAGGCGTGATGCGGAGC CCGTTAAGGGTGAAGTGGGTGATCCTGTGCTGTCGAGAAAAGCCTCTA GCGATGTTGATATTCGGCCCGTACCCTAAACCGACACAGGTAGTCAGGT AGAGAATACTAAGGCGTTCGGGTGAACTGTGGTTAAGGAACTCGGCA AAATGCCCCCGTAACTTCGGGAGAAGGGGGGCCACGGCGTGTGAACA ACTTTTCGTTGGGAGCGTGTTGTGGTCGCAGAGAATAGAGGGAAGCG ACTGTTTACTAAAAACACAGGTCCGTGCGAAGACGTTTAAGTTGATGT ATACGGACTGACGCCTGCCCGGTGCTGGAAGGTTAAGAGGACCGGTTA GTGACCTTTATGGTTGCGAAGCTGAGAATTTAAGCCCCAGTAAACGGC GGTGGTAACTATAACCATCCTAAGGTAGCGAAATTCCTTGTCGGGTAAG TTCCGACCTGCACGAATGGCGTAACGACTTCCCTGCTGTCTCAACCAC AGGCCCGGTGAAATTGCAGTACGAGTAAAGATGCTCGTTACGCGCGGC AGGACGAAAAGACCCCGGGACCTTCACTATAGCTTGGTATTGGTGTTT GATTCGGTTTGTGTAGGATAGGTGGGAGACTTCGATCATATGACGCTAG TTGTGTGTGAGTCGTTGGTGAAATACCACTCTGATCGGATTGGATGTCT TAACCTTGGCCCATGATCTGGGTTGGGGACAGTGCCTGGTGGGTAGTT TAACTGGGGCGGTTGCCTCCTAAAATGTAACGGAGGCGCCCAAAGGTT TCCTCAGCTTGGTTGGTAATCAGGTGGTGAGTGTAAGTGCACAAGGGA GCTTGACTGTGACACTGACAGGTGGAGCAGGGACGAAAGTCGGGACT AGTGATCCGGCACCTACTTGTGGTTGTGGTGTCGCTCAACGGATAAAA GGTACCCCGGGGATAACAGGCTGATCTTCCCCAAGAGTCCATATCGAC GGGATGGTTTGGCACCTCGATGTCGGCTCGTCGCATCCTGGGGCTGGA GTAGGTCCCAAGGGTTGGGCTGTTCGCCCATTAAAGCGGCACGCGAGC TGGGTTTAGAACGTCGTGAGACAGTTCGGTCTCTATCCGCCGCGCGCG TTGAAACTTGAAGGAAGGCTGTCCCTAGTACGAGAGGACCGGGACGG ACGTACCTCTGGTGTGCCAGTTGTTCCGCCAGGAGCAGGGCTGGTTGG CTACGTACGGGAGGGATAACCGCTGAAAGCATCTAAGCGGGAAGCCTG TTTCGAGATGAGGTTTCTTTTGAGGTTCCCTAGAGATTATGGGGTTGAT AGGCCAGATCTGGAAGCACTGTGAGGTGTGGAGGTGACTGGTACTAAT TTACCGATAACAACAAACCA |
| 28 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC |
| | ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCATCCTTATGTGGTGTGATGGCGTGCCTTTTGAAGAATGAGCCTGCGA GTCAGCGGCATGTCGCGAGGTTAACCCGTGTGGGGTAGCCGTAGGGAA ACCGAATCCTAACGAGGGTGATTTTAGTGGCATGTCTTGGACCCGAAG CGGAGTGATCTACCCATGGCCAGTGTGAAGCAGCTGTAAGAGGTTGTG GAGGCGCGAACCCACTTAGGTTGAAAACTGAGGGGATGAGTTGTGGG TAGGGGTGAAAGGCCAATCAAACTCCGTGATAGCTGGTTCTCCCCGAA ATGCATTTAGGTGCAGCGTCGTGTGTTTCTTGCCGGAGGTAGAGCTACT GGATGGTTTAGCGGGACCAACATCTTAGCGACATCAGCCAAACTCCGA ATGCCGGTAAGTCAGAGCACGGCAGTGAGACTGCGGGGGATAAGCTT CGTAGTCGAGAGGGAAACAGCCCAGATCGCCGGCTAAGGCCCCTAAG GGTGTGCTAAGTGGAAAAGGAGGTGGGGTCGCGAAGACAGCCAGGA GGTTGGCTTAGAAGCAGCCATCCTTGAAAGAGTGCGTAATAGCTCACT GGTCGAGTGATTCCGCGCCGACAATGTAGTGGGGCTTAAGTACACCGC CGAAGCCGCGGCAATGATCTTTATTGGATTGTTGGGTAGGGGAGCGTC GTGCATGCGTTGAAGCTTTGGGGTGACCTTGGGTGGAGTGTGTGCGAG TGAGAATGCAGGCATGAGTAACGAATGATGCGTGAGAAACGTATCCGC CGGATGACTAAGGGTTCCTGGGTCAAGTTAATCTTCCCAGGGTGAGTC GGGGCCTAAGGCGAGGCCGACAGGCGTAGTCGATGGATAACGGGTTG ATATTCCCGTACCCGAGTATGAGCGACCATGGTGAATCAGTGATACTAA CCACCCATAAGCACCCGCGAAAAGGCTTTGCTTTTTTGTGGTGTGTGG TTGCGTGGGACCTGATCTGGTAGTAGCTAAGTGATGGGGTGACGCAGG GAGGTAGCTCAGCCACTTATTGGATTGTGGTGTAAGCGTGTGGCACGC AGTGTAGGTCAAATCCGCACTGTTTTTGTGTGAGGCGTGATGCGGAGC CCGTAAAGGGTGAAGTGGGTGATCCTGTGCTGTCGAGAAAAGCCTCTA GCGATGTTGATATTCGGCCCGTACCCTAAACCGACACAGGTAGTCAGGT AGAGAATACTAAGGCGTTCGGGTGAACTGTGGTTAAGGAACTCGGCA AAATGCCCCCGTAACTTCGGGAGAAGGGGGGCCACGGCGTGTGAACA ACTTTTCGTTGGGAGCGTGTTGTGGTCGCAGAGAATAGAGGGAAGCG ACTGTTTACTAAAAACACAGGTCCGTGCGAAGACGTTTAAGTTGATGT ATACGGACTGACGCCTGCCCGGTGCTGGAAGGTTAAGAGGACCGGTTA GCCGTAAGGCGAAGCTGAGAATTTAAGCCCCAGTAAACGGCGGTGGTA ACTATAACCATCCTAAGGTAGCGAAATTCCTTGTCGGGTAAGTTCCGAC CTGCACGAATGGCGTAACGACTTCCCTGCTGTCTCAACCACAGGCCCG GTGAAATTGCAGTACGAGTAAAGATGCTCGTTACGCGCGGCAGGACGA AAAGACCCCGGGACCTTCACTATAGCTTGGTATTGGTGTTTGATTCGGT TTGTGTAGGATAGGTGGGAGACTTCGATCATATGACGCTAGTTGTGTGT GAGTCGTTGGTGAAATACCACTCTGATCGGATTGGATGTCTTAACCTTG GCCCATGATCTGGGTTGGGGACAGTGCCTGGTGGGTAGTTTAACTGGG GCGGTTGCCTCCTAAAATGTAACGGAGGCGCCCAAAGGTTTCCTCAGC TTGGTTGGTAATCAGGTGGTGAGTGTAAGTGCACAAGGGAGCTTGACT GTGACACTGACAGGTGGAGCAGGGACGAAAGTCGGGACTAGTGATCC GGCACCTACTTGTGGTTGTGGTGTCGCTCAACGGATAAAAGGTACCCC GGGGATAACAGGCTGATCTTCCCCAAGAGTCCATATCGACGGGATGGT TTGGCACCTCGATGTCGGCTCGTCGCATCCTGGGGCTGGAGTAGGTCC CAAGGGTTGGGCTGTTCGCCCATTAAAGCGGCACGCGAGCTGGGTTTA |
| | GAACGTCGTGAGACAGTTCGGTCTCTATCCGCCGCGCGCGTTGAAACT TGAAGGAAGGCTGTCCCTAGTACGAGAGGACCGGGACGGACGTACCT CTGGTGTGCCAGTTGTTCCGCCAGGAGCAGGGCTGGTTGGCTACGTAC GGGAGGGATAACCGCTGAAAGCATCTAAGCGGGAAGCCTGTTTCGAG ATGAGGTTTCTTTTGAGGTTCCCTAGAGATTATGGGGTTGATAGGCCAG ATCTGGAAGCACTGTGAGGTGTGGAGGTGACTGGTACTAATTTACCGA TAACAACAAACCA |
| 29 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCACCCCTTTGTGGTGTGATGGCGTGCCTTTTGAAGAATGAGCCTGCG AGTCAGCGGCATGTCGCGAGGTTAACCCGTGTGGGGTAGCCGTAGGGA AACCGAATCCTAACGAGGGTGATTTTAGTGGCATGTCTTGGACCCGAA GCGGAGTGATCTACCCATGGCCAGTGTGAAGCAGCTGTAAGAGGTTGT GGAGGCGCGAACCCACTTAGGTTGAAAACTGAGGGGATGAGTTGTGG GTAGGGGTGAAAGGCCAATCAAACTCCGTGATAGCTGGTTCTCCCCGA AATGCATTTAGGTGCAGCGTCGTGTGTTTCTTGCCGGAGGTAGAGCTAC TGGATGGTTTAGCGGGACCAACATCTTAGCGACATCAGCCAAACTCCG AATGCCGGTAAGTCAGAGCACGGCAGTGAGACTGCGGGGGATAAGCT TCGTAGTCGAGAGGGAAACAGCCCAGATCGCCGGCTAAGGCCCCTAA GGGTGTGCTAAGTGGAAAAGGAGGTGGGGTCGCGAAGACAGCCAGG AGGTTGGCTTAGAAGCAGCCATCCTTGAAAGAGTGCGTAATAGCTCAC TGGTCGAGTGATTCCGCGCCGACAATGTAGTGGGGCTTAAGTACACCG CCGAAGCCGCGGCAATGATCTTTATAGGATTGTTGGGTAGGGGAGCGT CGTGCATGCGTTGAAGCTTTGGGGTGACCTTGGGTGGAGTGTGTGCGA GTGAGAATGCAGGCATGAGTAACGAATGATGCGTGAGAAACGTATCCG CCGGATGACTAAGGGTTCCTGGGTCAAGTTAATCTTCCCAGGGTGAGT CGGGGCCTAAGGCGAGGCCGACAGGCGTAGTCGATGGATAACGGGTT GATATTCCCGTACCCGAGTATGAGCGACCATGGTGAATCAGTGATACTA ACCACCCATAAGCACCCGCGAAAAGGCTTTGCTTTTTTGTGGTGTGTG GTTGCGTGGGACCTGATCTGGTAGTAGCTAAGTGATGGGGTGACGCAG GGAGGTAGCTCAGCCACTTATTGGATTGTGGTGTAAGCGTGTGGCACG CAGTGTAGGTCAAATCCGCACTGTTTTTGTGTGAGGCGTGATGCGGAG CCCGTTAAGGGTGAAGTGGGTGATCCTGTGCTGTCGAGAAAAGCCTCT AGCGATGTTGATATTCGGCCCGTACCCTAAACCGACACAGGTAGTCAG GTAGAGAATACTAAGGCGTTCGGGTGAACTGTGGTTAAGGAACTCGGC AAAATGCCCCCGTAACTTCGGGAGAAGGGGGGCCACGGCGTGTGAAC AACTTTTCGTTGGGAGCGTGTTGTGGTCGCAGAGAATAGAGGGAAGC GACTGTTTACTAAAAACACAGGTCCGTGCGAAGACGTTTAAGTTGATG TATACGGACTGACGCCTGCCCGGTGCTGGAAGGTTAAGAGGACCGGTT AGTGACCTTTATGGTTGCGAAGCTGAGAATTTAAGCCCCAGTAAACGG CGGTGGTAACTATAACCATCCTAAGGTAGCGAAATTCCTTGTCGGGTAA GTTCCGACCTGCACGAATGGCGTAACGACTTCCCTGCTGTCTCAACCA CAGGCCCGGTGAAATTGCAGTACGAGTAAAGATGCTCGTTACGCGCGG CAGGACGAAAAGACCCCGGGACCTTCACTATAGCTTGGTATTGGTGTT TGATTCGGTTTGTGTAGGATAGGTGGGAGACTTCGATCATATGACGCTA GTTGTGTGTGAGTCGTTGGTGAAATACCACTCTGATCGGATTGGATGTC TTAACCTTGGCCCATGATCTGGGTTGGGGACAGTGCCTGGTGGGTAGT TTAACTGGGGCGGTTGCCTCCTAAAATGTAACGGAGGCGCCCAAAGGT TTCCTCAGCTTGGTTGGTAATCAGGTGGTGAGTGTAAGTGCACAAGGG AGCTTGACTGTGACACTGACAGGTGGAGCAGGGACGAAAGTCGGGAC TAGTGATCCGGCACCTACTTGTGGTTGTGGTGTCGCTCAACGGATAAA AGGTACCCCGGGGATAACAGGCTGATCTTCCCCAAGAGTCCATATCGA CGGGATGGTTTGGCACCTCGATGTCGGCTCGTCGCATCCTGGGGCTGG AGTAGGTCCCAAGGGTTGGGCTGTTCGCCCATTAAAGCGGCACGCGAG CTGGGTTTAGAACGTCGTGAGACAGTTCGGTCTCTATCCGCCGCGCGC GTTGAAACTTGAAGGAAGGCTGTCCCTAGTACGAGAGGACCGGGACG GACGTACCTCTGGTGTGCCAGTTGTTCCGCCAGGAGCAGGGCTGGTTG GCTACGTACGGGAGGGATAACCGCTGAAAGCATCTAAGCGGGAAGCCT GTTTCGAGATGAGGTTTCTTTTGAGGTTCCCTAGAGATTATGGGGTTGA TAGGCCAGATCTGGAAGCACTGTGAGGTGTGGAGGTGACTGGTACTAA TTTACCGATAACAACAAACCA |
| 30 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCACCCGTTTGTGGTGTGATGGCGTGCCTTTTGAAGAATGAGCCTGCG AGTCAGCGGCATGTCGCGAGGTTAACCCGTGTGGGGTAGCCGTAGGGA AACCGAATCCTAACGAGGGTGTTTTTAGTGGCATGTCTTGGACCCGAA GCGGAGTGATCTACCCATGGCCAGTGTGAAGCAGCTGTAAGAGGTTGT GGAGGCGCGAACCCACTTAGGTTGAAAACTGAGGGGATGAGTTGTGG GTAGGGGTGAAAGGCCAATCAAACTCCGTGATAGCTGGTTCTCCCCGA AATGCATTTAGGTGCAGCGTCGTGTGTTTCTTGCCGGAGGTAGAGCTAC TGGATGGTTTAGCGGGACCAACATCTTAGCGACATCAGCCAAACTCCG AATGCCGGTAAGTCAGAGCACGGCAGTGAGACTGCGGGGGATAAGCT TCGTAGTCGAGAGGGAAACAGCCCAGATCGCCGGCTAAGGCCCCTAA GGGTGTGCTAAGTGGAAAAGGAGGTGGGGTCGCGAAGACAGCCAGG AGGTTGGCTTAGAAGCAGCCATCCTTGAAAGAGTGCGTAATAGCTCAC TGGTCGAGTGATTCCGCGCCGACAATGTAGTGGGGCTTAAGTACACCG CCGAAGCCGCGGCAATGATCTTTATAGGATTGTTGGGTAGGGGAGCGT CGTGCATGCGTTGAAGCTTTGGGGTGACCTTGGGTGGAGTGTGTGCGA GTGAGAATGCAGGCATGAGTAACGAATGATGCGTGAGAAACGTATCCG CCGGATGACTAAGGGTTCCTGGGTCAAGTTAATCTTCCCAGGGTGAGT CGGGGCCTAAGGCGAGGCCGACAGGCGTAGTCGATGGATAACGGGTT GATATTCCCGTACCCGAGTATGAGCGACCATGGTGAATCAGTGATACTA ACCACCCATAAGCACCCGCGAAAAGGCTTTGCTTTTTTTGTGGTGTGTG GTTGCGTGGGACCTGATCTGGTAGTAGCTAAGTGATGGGGTGACGCAG GGAGGTAGCTCAGCCACTTATTGGATTGTGGTGTAAGCGTGTGGCACG CAGTGTAGGTCAAATCCGCACTGTTTTTGTGTGAGGCGTGATGCGGAG CCCGTTAAGGGTGAAGTGGGTGATCCTGTGCTGTCGAGAAAAGCCTCT AGCGATGTTGATATTCGGCCCGTACCCTAAACCGACACAGGTAGTCAG GTAGAGAATACTAAGGCGTTCGGGTGAACTGTGGTTAAGGAACTCGGC AAAATGCCCCCGTAACTTCGGGAGAAGGGGGGCCACGGCGTGTGAAC AACTTTTCGTTGGGAGCGTGTTGTGGTCGCAGAGAATAGAGGGAAGC GACTGTTTACTAAAAACACAGGTCCGTGCGAAGACGTTTAAGTTGATG TATACGGACTGACGCCTGCCCGGTGCTGGAAGGTTAAGAGGACCGGTT AGTGACCTTTATGGTTGCGAAGCTGAGAATTTAAGCCCCAGTAAACGG CGGTGGTAACTATAACCATCCTAAGGTAGCGAAATTCCTTGTCGGGTAA GTTCCGACCTGCACGAATGGCGTAACGACTTCCCTGCTGTCTCAACCA CAGGCCCGGTGAAATTGCAGTACGAGTAAAGATGCTCGTTACGCGCGG CAGGACGAAAAGACCCCGGGACCTTCACTATAGCTTGGTATTGGTGTT TGATTCGGTTTGTGTAGGATAGGTGGGAGACTTCGATCATATGACGCTA GTTGTGTGTGAGTCGTTGGTGAAATACCACTCTGATCGGATTGGATGTC TTAACCTTGGCCCATGATCTGGGTTGGGGACAGTGCCTGGTGGGTAGT TTAACTGGGGCGGTTGCCTCCTAAAATGTAACGGAGGCGCCCAAAGGT TTCCTCAGCTTGGTTGGTAATCAGGTGGTGAGTGTAAGTGCACAAGGG AGCTTGACTGTGACACTGACAGGTGGAGCAGGGACGAAAGTCGGGAC TAGTGATCCGGCACCTACTTGTGGTTGTGGTGTCGCTCAACGGATAAA AGGTACCCCGGGGATAACAGGCTGATCTTCCCCAAGAGTCCATATCGA CGGGATGGTTTGGCACCTCGATGTCGGCTCGTCGCATCCTGGGGCTGG AGTAGGTCCCAAGGGTTGGGCTGTTCGCCCATTAAAGCGGCACGCGAG CTGGGTTTAGAACGTCGTGAGACAGTTCGGTCTCTATCCGCCGCGCGC GTTGAAACTTGAAGGAAGGCTGTCCCTAGTACGAGAGGACCGGGACG GACGTACCTCTGGTGTGCCAGTTGTTCCGCCAGGAGCAGGGCTGGTTG GCTACGTACGGGAGGGATAACCGCTGAAAGCATCTAAGCGGGAAGCCT GTTTCGAGATGAGGTTTCTTTTGAGGTTCCCTAGAGATTATGGGGTTGA TAGGCCAGATCTGGAAGCACTGTGAGGTGTGGAGGTGACTGGTACTAA TTTACCGATAACAACAAACCAC |
| 31 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCACCCCTTTGTGGTGTGATGGCGTGCCTTTTGAAGAATGAGCCTGCG AGTCAGCGGCATGTCGCGAGGTTAACCCGTGTGGGGTAGCCGTAGGGA AACCGAATCCTAACGAGGGTGATTTTAGTGGCATGTCTTGGACCCGAA GCGGAGTGATCTACCCATGGCCAGTGTGAAGCAGCTGTAAGAGGTTGT GGAGGCGCGAACCCACTTAGGTTGAAAACTGAGGGGATGAGTTGTGG GTAGGGGTGAAAGGCCAATCAAACTCCGTGATAGCTGGTTCTCCCCGA AATGCATTTAGGTGCAGCGTCGTGTGTTTCTTGCCGGAGGTAGAGCTAC TGGATGGTTTAGCGGGACCAACATCTTAGCGACATCAGCCAAACTCCG AATGCCGGTAAGTCAGAGCACGGCAGTGAGACTGCGGGGGATAAGCT TCGTAGTCGAGAGGGAAACAGCCCAGATCGCCGGCTAAGGCCCCTAA GGGTGTGCTAAGTGGAAAAGGAGGTGGGGTCGCGAAGACAGCCAGG AGGTTGGCTTAGAAGCAGCCATCCTTGAAAGAGTGCGTAATAGCTCAC TGGTCGAGTGATTCCGCGCCGACAATGTAGTGGGGCTTAAGTACACCG CCGAAGCCGCGGCAATGATCTTTATAGGATTGTTGGGTAGGGGAGCGT CGTGCATGCGTTGAAGCTTTGGGGTGACCTTGGGTGGAGTGTGTGCGA GTGAGAATGCAGGCATGAGTAACGAATGATGCGTGAGAAACGTATCCG CCGGATGACTAAGGGTTCCTGGGTCAAGTTAATCTTCCCAGGGTGAGT CGGGGCCTAAGGCGAGGCCGACAGGCGTAGTCGATGGATAACGGGTT GATATTCCCGTACCCGAGTATGAGCGACCATGGTGAATCAGTGATACTA ACCACCCATAAGCACCCGCGAAAAGGCTTTGCTTTTTTGTGGTGTGT GGTTGCGTGGGACCTGATCTGGTAGTAGCTAAGTGATGGGGTGACGCA GGGAGGTAGCTCAGCCACTTATTGGATTGTGGTGTAAGCGTGTGGCAC GCAGTGTAGGTCAAATCCGCACTGTTTTTGTGTGAGGCGTGATGCGGA GCCCGTTAAGGGTGAAGTGGGTGATCCTGTGCTGTCGAGAAAAGCCTC TAGCGATGTTGATATTCGGCCCGTACCCTAAACCGACACAGGTAGTCAG GTAGAGAATACTAAGGCGTTCGGGTGAACTGTGGTTAAGGAACTCGGC AAAATGCCCCCGTAACTTCGGGAGAAGGGGGGCCACGGCGTGTGAAC AACTTTTCGTTGGGAGCGTGTTGTGGTCGCAGAGAATAGAGGGAAGC GACTGTTTACTAAAAACACAGGTCCGTGCGAAGACGTTTAAGTTGATG TATACGGACTGACGCCTGCCCGGTGCTGGAAGGTTAAGAGGACCGGTT AGCCGTAAGGCGAAGCTGAGAATTTAAGCCCCAGTAAACGGCGGTGG TAACTATAACCATCCTAAGGTAGCGAAATTCCTTGTCGGGTAAGTTCCG ACCTGCACGAATGGCGTAACGACTTCCCTGCTGTCTCAACCACAGGCC CGGTGAAATTGCAGTACGAGTAAAGATGCTCGTTACGCGCGGCAGGAC GAAAAGACCCCGGGACCTTCACTATAGCTTGGTATTGGTGTTTGATTCG GTTTGTGTAGGATAGGTGGGAGACTTCGATCATATGACGCTAGTTGTGT GTGAGTCGTTGGTGAAATACCACTCTGATCGGATTGGATGTCTTAACCT TGGCCCATGATCTGGGTTGGGGACAGTGCCTGGTGGGTAGTTTAACTG GGGCGGTTGCCTCCTAAAATGTAACGGAGGCGCCCAAAGGTTTCCTCA GCTTGGTTGGTAATCAGGTGGTGAGTGTAAGTGCACAAGGGAGCTTGA CTGTGACACTGACAGGTGGAGCAGGGACGAAAGTCGGGACTAGTGAT CCGGCACCTACTTGTGGTTGTGGTGTCGCTCAACGGATAAAAGGTACC CCGGGGATAACAGGCTGATCTTCCCCAAGAGTCCATATCGACGGGATG GTTTGGCACCTCGATGTCGGCTCGTCGCATCCTGGGGCTGGAGTAGGT CCCAAGGGTTGGGCTGTTCGCCCATTAAAGCGGCACGCGAGCTGGGTT TAGAACGTCGTGAGACAGTTCGGTCTCTATCCGCCGCGCGCGTTGAAA CTTGAAGGAAGGCTGTCCCTAGTACGAGAGGACCGGGACGGACGTAC CTCTGGTGTGCCAGTTGTTCCGCCAGGAGCAGGGCTGGTTGGCTACGT ACGGGAGGGATAACCGCTGAAAGCATCTAAGCGGGAAGCCTGTTTCG AGATGAGGTTTCTTTTGAGGTTCCCTAGAGATTATGGGGTTGATAGGCC AGATCTGGAAGCACTGTGAGGTGTGGAGGTGACTGGTACTAATTTACC GATAACAACAAACCAC |
| 32 | TTTGTTGTTTTGTAGGGCACACGGTGGATGCCTTGGCATATCAAGCCGA TGAAGGACGTGAGAGGCTGCGTTATGCCTCGGGGAGTTGCCAACTAAG |
| | CGTTGATCCGAGGATGTCCGAATGGGGAAACCCAGCCGCAGTGATGTG TGGTTACCTGCCAGTGAATGTATAGCTGGTGTGGAGGTTGACACGGGG AAGTGAAACATCTCAGTACCCGTAGGAGAAGAAAACAATTGTGATTCC GTTAGTAGTGGCGAGCGAACGTGGATGATGGCTAAAACTTATGTGTGT GATACCCGGCAGGGGTTGCATGTAGGTGGTTGTGGGGCAATGACGTTC ATATTCTGCCGGATGTGGGCATGTGCTGCGTGGTTAGTGGAAGTGGTGT GGAAACACCTGCCGTAGAAGGTGAGAGTCCTGTACACGAAGATCATG GTGGTGTGTGTGGTTGTTGATACCCCGAGTAGCAGCGGGCTCGTGGAA TCTGCTGTGAATTAGCCGGGACCACCCGGTAAGCCTGAATACTTGATAT GACCGATAGCGGATTAGTACCGTGAGGGAATGGTGAAAAGTACCCCGG GAGGGGAGTGAAATAGTACCTGAAACCGTGTGCTGTCAATCCGTCAGA GCACCCCTTTGTGGTGTGATGGCGTGCCTTTTGAAGAATGAGCCTGCG AGTCAGCGGCATGTCGCGAGGTTAACCCGTGTGGGGTAGCCGTAGGGA AACCGAATCCTAACGAGGGTGATTTTAGTGGCATGTCTTGGACCCGAA GCGGAGTGATCTACCCATGGCCAGTGTGAAGCAGCTGTAAGAGGTTGT GGAGGCGCGAACCCACTTAGGTTGAAAACTGAGGGGATGAGTTGTGG GTAGGGGTGAAAGGCCAATCAAACTCCGTGATAGCTGGTTCTCCCCGA AATGCATTTAGGTGCAGCGTCGTGTGTTTCTTGCCGGAGGTAGAGCTAC TGGATGGTTTAGCGGGACCAACATCTTAGCGACATCAGCCAAACTCCG AATGCCGGTAAGTCAGAGCACGGCAGTGAGACTGCGGGGGATAAGCT TCGTAGTCGAGAGGGAAACAGCCCAGATCGCCGGCTAAGGCCCCTAA GGGTGTGCTAAGTGGAAAAGGAGGTGGGGTCGCGAAGACAGCCAGG AGGTTGGCTTAGAAGCAGCCATCCTTGAAAGAGTGCGTAATAGCTCAC TGGTCGAGTGATTCCGCGCCGACAATGTAGTGGGGCTTAAGTACACCG CCGAAGCCGCGGCAATGATCTTTATAGGATTGTTGGGTAGGGGAGCGT CGTGCATGCGTTGAAGCTTTGGGGTGACCTTGGGTGGAGTGTGTGCGA GTGAGAATGCAGGCATGAGTAACGAATGATGCGTGAGAAACGTATCCG CCGGATGACTAAGGGTTCCTGGGTCAAGTTAATCTTCCCAGGGTGAGT CGGGGCCTAAGGCGAGGCCGACAGGCGTAGTCGATGGATAACGGGTT GATATTCCCGTACCCGAGTATGAGCGACCATGGTGAATCAGTGATACTA ACCACCCATAAGCACCCGCGAAAAGGCTTTGCTTTTTTGTGGTGTGT GGTTGCGTGGGACCTGATCTGGTAGTAGCTAAGTGATGGGGTGACGCA GGGAGGTAGCTCAGCCACTTATTGGATTGTGGTGTAAGCGTGTGGCAC GCAGTGTAGGTCAAATCCGCACTGTTTTTGTGTGAGGCGTGATGCGGA GCCCGTTAAGGGTGAAGTGGGTGATCCTGTGCTGTCGAGAAAAGCCTC TAGCGATGTTGATATTCGGCCCGTACCCTAAACCGACACAGGTAGTCAG GTAGAGAATACTAAGGCGTTCGGGTGAACTGTGGTTAAGGAACTCGGC AAAATGCCCCCGTAACTTCGGGAGAAGGGGGGCCACGGCGTGTGAAC AACTTTTCGTTGGGAGCGTGTTGTGGTCGCAGAGAATAGAGGGAAGC GACTGTTTACTAAAAACACAGGTCCGTGCGAAGACGTTTAAGTTGATG TATACGGACTGACGCCTGCCCGGTGCTGGAAGGTTAAGAGGACCGGTT AGTGACCTTTATGGTTGCGAAGCTGAGAATTTAAGCCCCAGTAAACGG CGGTGGTAACTATAACCATCCTAAGGTAGCGAAATTCCTTGTCGGGTAA GTTCCGACCTGCACGAATGGCGTAACGACTTCCCTGCTGTCTCAACCA CAGGCCCGGTGAAATTGCAGTACGAGTAAAGATGCTCGTTACGCGCGG CAGGACGAAAAGACCCCGGGACCTTCACTATAGCTTGGTATTGGTGTT TGATTCGGTTTGTGTAGGATAGGTGGGAGACTTCGATCATATGACGCTA GTTGTGTGTGAGTCGTTGGTGAAATACCACTCTGATCGGATTGGATGTC TTAACCTTGGCCCATGATCTGGGTTGGGGACAGTGCCTGGTGGGTAGT TTAACTGGGGCGGTTGCCTCCTAAAATGTAACGGAGGCGCCCAAAGGT TTCCTCAGCTTGGTTGGTAATCAGGTGGTGAGTGTAAGTGCACAAGGG |
| | AGCTTGACTGTGACACTGACAGGTGGAGCAGGGACGAAAGTCGGGAC TAGTGATCCGGCACCTACTTGTGGTTGTGGTGTCGCTCAACGGATAAA AGGTACCCCGGGGATAACAGGCTGATCTTCCCCAAGAGTCCATATCGA CGGGATGGTTTGGCACCTCGATGTCGGCTCGTCGCATCCTGGGGCTGG AGTAGGTCCCAAGGGTTGGGCTGTTCGCCCATTAAAGCGGCACGCGAG CTGGGTTTAGAACGTCGTGAGACAGTTCGGTCTCTATCCGCCGCGCGC GTTGAAACTTGAAGGAAGGCTGTCCCTAGTACGAGAGGACCGGGACG GACGTACCTCTGGTGTGCCAGTTGTTCCGCCAGGAGCAGGGCTGGTTG GCTACGTACGGGAGGGATAACCGCTGAAAGCATCTAAGCGGGAAGCCT GTTTCGAGATGAGGTTTCTTTTGAGGTTCCCTAGAGATTATGGGGTTGA TAGGCCAGATCTGGAAGCACTGTGAGGTGTGGAGGTGACTGGTACTAA TTTACCGATAACAACAAACCAC |

### EXAMPLE 4: Construction of an L-arginine-producing strain introduced with a mutant promoter

### Example 4-1: Construction of a recombinant vector for introducing a mutant promoter

To evaluate the effects of the protein variant identified in the above Example 3, a vector capable of introducing it onto a chromosome was prepared.

Specifically, in order to introduce each of the BBD29_00030, BBD29_05930, BBD29_08260, BBD29_02985, BBD29_06380, BBD29_07445, BBD29_12405, BBD29_07610, and BBD29_04520 gene mutations into a CJR100 strain, vectors comprising target mutations were prepared.

Specifically, the genomic DNA of the CJR100_mt3 strain was extracted using a G-spin Total DNA Extraction Mini Kit (Intron, Cat. No. 17045) according to the protocol provided in the kit, and PCR was performed using the genomic DNA as a template. SolgTM Pfu-X DNA polymerase was used as the polymerase, and the PCR conditions were as follows: denaturation at 95°C for 4 minutes; 27 cycles of denaturation at 95°C for 30 seconds, annealing at 60°C for 30 seconds, and polymerization at 72°C for 50 seconds; and polymerization was performed at 72°C for 5 minutes. The sequences of the primer pairs used in the experiment are shown in Table 8 below.

The above-obtained mutation introduction fragment and the pDC24 vector (SEQ ID NO: 81) treated with the restriction enzyme SmaI were cloned using the Gibson assembly method (DG Gibson et al., NATURE METHODS, VOL.6 NO.5, MAY 2009, NEBuilder HiFi DNA Assembly Master Mix) to obtain recombinant plasmids, and the vectors comprising each mutation-introduced fragment were named pDC24-BBD29_00030*; pDC24-BBD29_05930*; pDC24-BBD29_08260*; pDC24-BBD29_02985*; pDC24-BBD29_06380*; pDC24-BBD29_07445*; pDC24-BBD29_12405*; pDC24-BBD29_07610*; and pDC24-BBD29_04520*.

**[Table 8]**

| Name | SEQUENCE (5'-> 3') | SEQ ID NO |
|---|---|---|
| BBD29_00030*_F | tgaattcgagctcggtacccTGAAAACTCAGTCGATGCCC | 33 |
| BBD29_00030*_R | gtcgactctagaggatccccGTTATCGCATCCCAGAAACC | 34 |
| BBD29_05930*_F | tgaattcgagctcggtacccGGACCCTGTCTAAACTTCGT | 35 |
| BBD29_05930*_R | gtcgactctagaggatccccTCACCAACGGACCCGTCGGC | 36 |
| BBD29_08260*_F | tgaattcgagctcggtacccCGGATCCGCGTCTTCATCAG | 37 |
| BBD29_08260*_R | gtcgactctagaggatccccCAGACCGAGGCTGCAATTGC | 38 |
| BBD29_02985*_F | tgaattcgagctcggtacccCCGGGGTCATTGCCCGATCC | 39 |
| BBD29_02985*_R | gtcgactctagaggatccccGACGCCAAGCGGGAGTCGAC | 40 |
| BBD29_06380*_F | tgaattcgagctcggtacccTGTGGAACAACAACCCCCGA | 41 |
| BBD29_06380*_R | gtcgactctagaggatccccGCACTTAATGATCCGAATCT | 42 |
| BBD29_07445*_F | tgaattcgagctcggtacccGCGGACTTCTTTGAGCCCAT | 43 |
| BBD29_07445*_R | gtcgactctagaggatccccACGCGCCCGGCGCCCTTTGG | 44 |
| BBD29_12405*_F | tgaattcgagctcggtacccACGCATGCACGACGCTCCCC | 45 |
| BBD29_12405*_R | gtcgactctagaggatccccCATGTGCTGCGTGGTTAGTG | 46 |
| BBD29_07610*_F | tgaattcgagctcggtacccCTTATGTGTGTGATACCCGG | 47 |
| BBD29_07610*_R | gtcgactctagaggatccccCTCAGCTTCGCCTTACGGCT | 48 |
| BBD29_04520*_F | tgaattcgagctcggtacccACAATGTAGTGGGGCTTAAG | 49 |
| BBD29_04520*_R | gtcgactctagaggatccccTTGTTCACACGCCGTGGCCC | 50 |

### EXAMPLE 4-2: Construction of an L-arginine-producing strain introduced with a mutant gene

The 9 types of vectors prepared in the above Example 4-1 were transformed into the arginine-producing strain CJR100 by an electroporation method, and strains in which the vector was inserted onto a chromosome by recombination of a homologous sequence were selected using a kanamycin medium. Thereafter, for the transformants in which the second recombination was completed, a strain into which a variant promoter was introduced was confirmed through PCR using the primer pairs of Table 9 below.

**[Table 9]**

| Name | SEQUENCE (5'-> 3') | SEQ ID NO |
|---|---|---|
| BBD29_00030*_CF | GTTACGCGCCGCAGGAGC | 51 |
| BBD29_00030*_CR | AGCAGTGTGCACACCAAC | 52 |
| BBD29_05930*_CF | TGCGAGCCAGGTGGTCGC | 53 |
| BBD29_05930*_CR | CGGGCTCAAACGGCCTCA | 54 |
| BBD29_08260*_CF | CAACTGGCTCATGGCGCG | 55 |
| BBD29_08260*_CR | GATCTCGATGATTCTCAA | 56 |
| BBD29_02985*_CF | CACCGGGAAGTACTTTAC | 57 |
| BBD29_02985*_CR | TCTGGCGGTGGGCATCCT | 58 |
| BBD29_06380*_CF | AAGCTATCTCGCGACGGT | 59 |
| BBD29_06380*_CR | CTACCGCTGGACCGAGTC | 60 |
| BBD29_07445*_CF | CGGTTACAAGCCTGGCCG | 61 |
| BBD29_07445*_CR | AGCATTGCTGATCTTGGT | 62 |
| BBD29_12405*_CF | AAGCCTTTTCGCGGGTGC | 63 |
| BBD29_12405*_CR | AGTGAATGTATAGCTGGT | 64 |
| BBD29_07610*_CF | GTCAACACGGGTAGAGAA | 65 |
| BBD29_07610*_CR | TCACCAACGACTCACACA | 66 |
| BBD29_04520*_CF | TGTGTTTCTTGCCGGAGG | 67 |
| BBD29_04520*_CR | GGAAGTCGTTACGCCATT | 68 |

The recombinant strains were named as CJR100△BBD29_00030::BBD29_00030*; CJR100△BBD29_05930::BBD29_05930*; CJR100△BBD29_08260::BBD29_08260*; CJR100△BBD29_02985::BBD29_02985*; CJR100△BBD29_06380::BBD29_06380*; CJR100△BBD29_07445::BBD29_07445*; CJR100△BBD29_12405::BBD29_12405*; CJR100△BBD29_07610::BBD29_07610*; and CJR100△BBD29_04520::BBD29_04520*, respectively.

### EXMPLE 5. Evaluation of L-arginine production ability of the L-arginine-producing strain introduced with a mutant promoter

To evaluate the L-arginine production ability of the recombinant strains constructed in Example 4-2, they were cultured and evaluated using the following method.

Each strain was inoculated into a 250-ml corner-baffle flask containing 25 ml of seed medium and cultured at 30°C for 20 hours with shaking at 200 rpm. One ml of the seed culture was inoculated into a 250-ml corner-baffle flask containing 24 ml of production medium and cultured at 30°C for 54 hours with shaking at 200 rpm. The medium composition was the same as in Example 2-2, and the experiment was repeated three times.

After the culture was completed, the amount of L-arginine produced was measured using high-performance liquid chromatography (HPLC), and the average value of the analysis results is shown in Table 10 below.

**[Table 10]**

| Strain name | L-arginine concentration (g/L) |
|---|---|
| CJR100 | 5.9 |
| CJR100ΔBBD29_00030::BBD29_00030* | 6.01 |
| CJR100ΔBBD29_05930::BBD29_05930* | 6.12 |
| CJR100ΔBBD29_08260::BBD29_08260* | 6.04 |
| CJR100ΔBBD29_02958::BBD29_02958* | 6.01 |
| CJR100ΔBBD29_06380::BBD29_06380* | 5.98 |
| CJR100ΔBBD29_07445::BBD29_07445* | 6.26 |
| CJR100ΔBBD29_12405::BBD29_12405* | 6.01 |
| CJR100ΔBBD29_07610::BBD29_07610* | 5.99 |
| CJR100ΔBBD29_04520::BBD29_04520* | 6 |

As a result, as shown in Table 10, it was confirmed that most of the arginine-producing strains expressing the protein variant have an arginine-producing ability at a level equivalent to the parent strain or superior to the parent strain. In particular, it was confirmed that the arginine-producing ability of the CJR100ΔBBD29_07445::BBD29_07445* strain was significantly increased compared to the parent strain CJR100.

Through this, it was confirmed that L-arginine can be more efficiently produced by introducing a protein variant in which the 922nd amino acid of the protein (excinuclease ABC subunit A) encoded by the BBD29_07445 gene is substituted from glycine (Gly, G) to glutamic acid (Glu, E).

From the above description, those skilled in the art to which the present disclosure pertains will be able to understand that the present disclosure may be practiced in other specific forms without changing its technical spirit or essential features. In this regard, it should be understood that the embodiments described above are illustrative in all respects and not restrictive. The scope of the present disclosure should be interpreted such that all changes or modifications derived from the meaning and scope of the following claims and their equivalent concepts, rather than the above detailed description, are included in the scope of the present disclosure.

## Claims

1. A protein variant comprising an amino acid sequence wherein an amino acid corresponding to position 922 from the N-terminus in the amino acid sequence of SEQ ID NO: 74 is substituted with glutamic acid.

2. The protein variant according to claim 1, wherein the protein variant has an amino acid sequence identity of 90% or more and less than 100% with the amino acid sequence of SEQ ID NO: 74.

3. The protein variant according to claim 1, wherein the protein variant comprises the amino acid sequence of SEQ ID NO: 20.

4. A polynucleotide encoding the protein variant of claim 1.

5. A microorganism of the genus *Corynebacterium* comprising at least one selected from the group consisting of the protein variant of claim 1 and a polynucleotide encoding the protein variant.

6. The microorganism according to claim 5, wherein the microorganism of the genus *Corynebacterium* is *Corynebacterium glutamicum.*

7. The microorganism according to claim 5, wherein the microorganism has increased L-arginine production ability, compared to a microorganism of genus *Corynebacterium* that does not comprise at least one selected from the group consisting of the protein variant and the polynucleotide encoding the protein variant.

8. A method for producing L-arginine, comprising culturing the microorganism of claim 5 in a medium.

9. The method for producing L-arginine according to claim 8, wherein the method further comprises recovering L-arginine from the cultured medium or the cultured microorganism.

10. A use of the microorganism of any one of claims 5 to 7, for producing L-arginine.

11. A composition, method, product, process, or use **characterized by** one or more elements disclosed in the present disclosure.
